# EUROPEAN PATENT SPECIFICATION

(11) **EP 0 982 590 B1**
(45) Date of publication and mention of the grant of the patent: **19.11.2003**
(21) Application number: 99112732.5
(22) Date of filing: 01.07.1999
(51) Int. Cl.: G01N 33/546, G01N 33/558, C12Q 1/28, C12Q 1/42, C12Q 1/68, G01N 33/569

(54) **Labeled conjugate and detection method using the same**
Markiertes Kojugat und Nachweismethode unter Verwendung desselben
Conjugué marqué et méthode de détection utilisant ce conjugué

(30) Priority: 01.07.1998 JP 18615998; 03.08.1998 JP 21925098; 09.10.1998 JP 28798998
(43) Date of publication of application: 01.03.2000
(73) Proprietor: NITTO DENKO CORPORATION, Osaka (JP)
(72) Inventor: Okada, Kenichi, Ibaraki-shi, Osaka (JP); Tatsumi, Motoshige, Ibaraki-shi, Osaka (JP); Kitaura, Chieko, Ibaraki-shi, Osaka (JP); Senda, Shuji, Ibaraki-shi, Osaka (JP); Saika, Takeshi, Ibaraki-shi, Osaka (JP); Okada, Keisaku, Ibaraki-shi, Osaka (JP)
(74) Representative: VOSSIUS & PARTNER

(56) References cited:
- EP-A- 0 174 195
- EP-A- 0 811 847
- WO-A-93/15230
- US-A- 5 266 497
- US-A- 5 310 650
- DATABASE WPI, week 199203, Derwent Publications Ltd., London, GB; Class B04, AN 1992-020459, XP002900737 & JP 03 269362 A (TOYO INK MFG CO),

## Description

The present invention relates to a labeled conjugate comprising a labeled immunochemical component suitable for immunoassays, more specifically to an enzyme-labeled specific conjugate, wherein a specific binding substance and an enzyme are immobilized onto a carrier, a method for detection of an analyte using the enzyme-labeled specific conjugate, and a test strip as defined in appended claims 1-26.

As one of methods for identifying and quantifying an antigen or antibody utilizing high specificity and detection sensitivity of an antigen-antibody reaction, there has been known a labeled immunoassay using an antigen or antibody labeled with a substance which is readily separated and detected. A labeled antigen or antibody used in this method is obtained generally by binding a label to an antigen or antibody (hereinafter referred to as "labeled antigen" or "labeled antibody," respectively). Such binding has conventionally been performed by means of crosslinking. However, a conventional method involves crosslinking of antigens or antibodies with each other, or crosslinking of labels with each other, so that a ratio which does not contribute to the production of a labeled antigen or a labeled antibody becomes high. Accordingly, the production yield is low, and the binding density of the label on the antigen or antibody also becomes markedly low, so that its function as a label is lowered. In addition, the amount of the label capable of linking to an antigen or antibody depends on the molecular size of the label. In a conventional method employing a direct linking of a label to an antigen or antibody, there is a disadvantage that the activity, functionality and specificity of the antigen or the antibody are likely to be lost.

Furthermore, since it is impossible to determine a plurality of items simultaneously, it is required to determine with a plurality of kits arranged in parallel.

On the other hand, the detection of a pathogenic microorganism in a food or from an individual, such as *E. coli* O157 which has been recently a great concern, may be time-consuming and require complicated procedures. In addition, a more rapid diagnosis may be required in some diseases. For such diagnosis, various immunoassays such as a labeled immunoassay described above are employed appropriately. Recently, an immunochromatography has been remarked with an interest since it may allow a rapid and simple immunoassay procedure. This method involves, for instance, a process described below. An antigen such as a bacterial cell is added to a water-absorbent substrate onto which an antibody (first antibody) is applied, and then wicked therethrough, thereby allowing the first antibody to capture the antigen. Subsequently, a labeled antibody (second antibody) which binds specifically to the antigen is added to the water-absorbent substrate, and then wicked, thereby binding the antigen which has already been captured to the antibody. Finally, an immunological complex (first antibody-antigen-second antibody-label) formed at the antibody application site on the water-absorbent substrate is visually confirmed. Besides the above, EIA (enzyme immunoassay) or a method employing a probe is also employed, but no method which is satisfactorily simple, rapid and/or sensitive has yet been established.

JP-A-32 69 362 discloses an immunoassay reagent consisting of water dispersible latex particles having an antibody and an enzyme immobilized on their surface, whereby the enzyme is a hydrolytic enzyme which function is to lyse the cells for releasing intracellular material. WO-A-93/15230 discloses a detection method based on the use of a test strip, which comprises binding in the capture region a soluble complex between a conjugate consisting of a latex particle having an enzyme-labeled specific member attached thereto and the analyte to an unlabeled specific binding member attached to the porous strip.

An object of the present invention is to provide a labeled conjugate comprising a labeled immunochemical component suitable for simple, rapid and highly sensitive immunoassays.

Another object of the present invention is to provide a method for detecting an analyte using an enzyme-labeled specific conjugate, such as a labeled conjugate, and a test strip.

These and other objects of the present invention will be apparent from the following description.

As a result of intensive studies in view of the above problems, the present inventors have succeeded in finding a novel method for detecting an analyte which is excellent in simplicity, sensitivity and rapidness by the use of an enzyme-labeled specific conjugate which is a labeled conjugate in which an enzyme as a label is linked via a carrier, to a specific binding substance, and a test strip used therefor.

Accordingly, the present invention pertains to:
[1] a labeled conjugate comprising water-dispersible polymeric particles as a carrier, at least one immunochemical component selected from the group consisting of antigens, haptens, antibodies, and oligonucleotides, and an enzyme, wherein the immunochemical component and the enzyme are immobilized onto a surface of the carrier, and wherein a total amount of the immunochemical component and the enzyme immobilized is from 5 to 200 mg per 1 g of the water-dispersible polymeric particles on a dry basis;
[2] a method for detecting an analyte, comprising the steps of:
   (1) forming a complex of:
      (a) an enzyme-labeled specific conjugate resulting from linking via a carrier an enzyme and a first specific binding substance capable of specifically binding to an analyte; and
      (b) the analyte,
      to wick the resulting complex on a water-absorbent substrate;
   (2) capturing the complex at a capture region immobilized on the water-absorbent substrate with a second specific binding substance capable of specifically binding to the analyte; and
   (3) detecting the captured complex;
[3] a method for detecting a plurality of analytes, comprising the steps of:
   (1) forming a complex or complexes of:
      (a) an enzyme-labeled specific conjugate resulting from linking, directly or via a carrier, an enzyme and a first specific binding substance capable of specifically binding to an analyte; and
      (b) a plurality of different analytes,
      to wick the resulting complex or complexes on a water-absorbent substrate;
   (2) capturing the complex or complexes at the plurality of capture regions on the water-absorbent substrate, each of which is immobilized with a second specific binding substance capable of specifically binding to one of the plurality of different analytes; and
   (3) detecting the captured complex or complexes;
[4] a test strip comprising:
   (a) a capture region arranged on a water-absorbent substrate, comprising a second specific binding substance capable of specifically binding to an analyte, wherein the second specific binding substance is immobilized thereon;
   (b) a dried reagent region comprising an enzyme-labeled specific conjugate resulting from linking via a carrier an enzyme and a first specific binding substance capable of specifically binding to the analyte, wherein the dried reagent region is arranged on the water-absorbent substrate such that the enzyme-labeled specific conjugate leaves and wicks therefrom when contacted with water; and
   (c) a sample receiver arranged at a position upstream of the dried reagent region or the same position as the dried reagent region.
[5] A test strip comprising:
   (a) a plurality of capture regions arranged on a water-absorbent substrate, each comprising a second specific binding substance capable of specifically binding to one of a plurality of different analytes, wherein each of the second specific binding substances is immobilized thereon;
   (b) a dried reagent region comprising an enzyme-labeled specific conjugate resulting from linking via a carrier, an enzyme and first specific binding substances, each of which is capable of specifically binding to one of the plurality of analytes to form a complex, wherein the dried reagent region is arranged on the water-absorbent substrate such that the enzyme-labeled specific conjugate leaves and wicks therefrom when contacted with water; and
   (c) a sample receiver arranged at a position upstream of the dried reagent region or the same position as the dried reagent region;
[6] a test kit of an analyte comprising:
   (1) a test strip comprising a capture region arranged on a water-absorbent substrate, comprising a second specific binding substance capable of specifically binding to the analyte, and a sample receiver arranged at a position upstream of the capture region; and
   (2) an enzyme-labeled specific conjugate resulting from linking via a carrier, an enzyme and a first specific binding substance capable of specifically binding to the analyte; and
[7] a test kit of a plurality of analytes comprising:
   (1) a test strip comprising a plurality of capture regions arranged on a water-absorbent substrate, each comprising a second specific binding substance capable of specifically binding to one of the plurality of different analytes, and a sample receiver arranged at a position upstream of the capture regions; and
   (2) an enzyme-labeled specific conjugate resulting from linking via a carrier, an enzyme and a first specific binding substance capable of specifically binding to the analyte.

According to the method for detection or a test strip using an enzyme-labeled specific conjugate of the present invention, since a specific binding substance and an enzyme with an active state are immobilized in specified amounts on the surface of a carrier, immunoassay can be performed with high sensitivity and high accuracy, so that there can be detected one analyte or a plurality of different analytes. Moreover, this enzyme-labeled specific conjugate can readily be produced and purified.

The present invention will become more fully understood from the detailed description given hereinbelow and the accompanying drawings, and wherein:
Figure 1 is a schematic view showing one embodiment of the present invention;
Figure 2 is a schematic view showing another embodiment of the present invention;
Figure 3 is a schematic view showing still another embodiment of the present invention;
Figure 4 is a schematic view showing still another embodiment of the present invention; and
Figure 5 is a schematic view showing still another embodiment of the present invention.

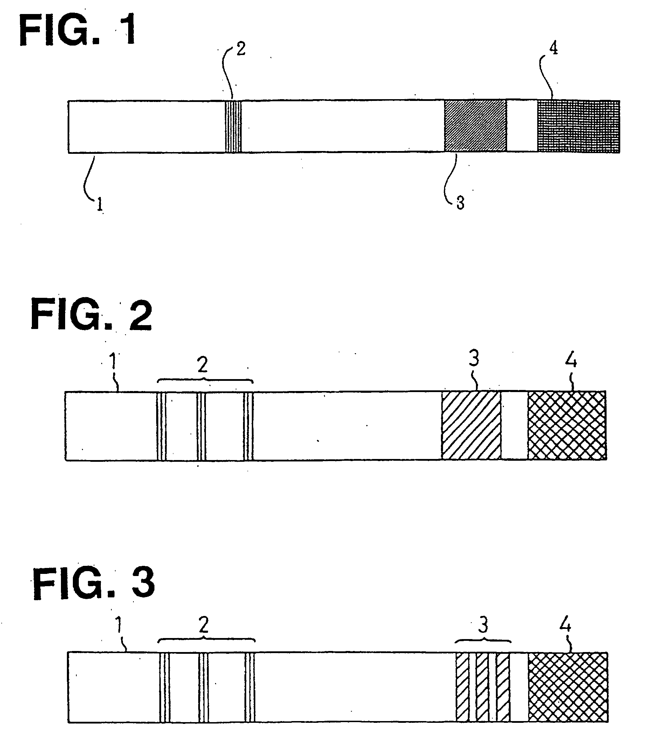

The reference numerals in each of Figures 1 to 5 are as follows:
1 is a water-absorbent substrate, 2 a capture region, 3 a dried reagent region, and 4 a sample receiver.

The method for detection according to the present invention is characterized in the use of an enzyme-labeled specific conjugate resulting from linking via a carrier, an enzyme and a first specific binding substance capable of specifically binding to an analyte.

The carrier used in the enzyme-labeled specific conjugate is not particularly limited as long as it is capable of linking a specific binding substance and an enzyme in the present invention on its surface. The carrier usually includes metal colloids, water-dispersible polymeric particles, silicones, silicas, glass, diatomaceous earth, and the like. Among them, the metal colloids and the water-dispersible polymeric particles are preferred.

The metal colloids include, for instance, a gold colloid and a selenium colloid.

The water-dispersible polymeric particles are prepared by emulsion polymerization of one or more monomers each having an unsaturated double bond. The usable monomer includes, for instance, olefinic monomers such as ethylene and propylene; vinyl monomers such as vinyl acetate and vinyl chloride; styrenic monomers such as styrene, methylstyrene and chlorostyrene; methacrylic acid ester monomers such as methyl acrylate; diene monomers such as butadiene, and the like.

The water-dispersible polymeric particles used in the present invention include, in addition to homopolymers or copolymers of the monomers described above, those copolymerized with a modifier monomer, for the purpose of giving the resulting particles with a functional group or an ionic group or of increasing the dispersion stability of the particles in an aqueous medium. The modifier monomer includes acrylic acid, methacrylic acid, fluorinated methacrylic acid esters such as 2,2,2-trifluoroethyl methyl methacrylate, acrylonitrile, methacrylonitrile, acrylamide, sodium salt of styrene sulfonate, sodium salt of sulfopropyl (meth)acrylate, N-vinyl-2-pyrrolidone, 2-hydroxyethyl acrylate, 2-hydroxyethyl methacrylate, glycidyl methacrylate, and the like.

In addition, in the present invention, various commercially available water-dispersible polymeric particles can also be favorably used. The commercially available water-dispersible polymeric particles include, for instance, emulsions comprising homopolymers and copolymers of styrene and its derivatives, for example, p-chlorostyrene, as well as various styrenic copolymers such as styrene-butadiene copolymers and styrene-acrylonitrile-butadiene copolymers and the like. Also, there can be used in the present invention homopolymers of long chain alkyl esters of (meth)acrylic acid or derivatives thereof as well as copolymers thereof with methyl (meth)acrylate, ethyl (meth)acrylate, glycidyl (meth)acrylate, or the like. Copolymers of styrene or its derivatives mentioned above with a (meth)acrylic acid ester or its derivatives can also be used. Also exemplified are rubbers, nylons, polyurethanes, microcrystalline celluloses, and the like.

Among the water-dispersible polymeric particles mentioned above, a polymer of which main constituent is a styrenic monomer is preferred from the viewpoint of its particle stability, and it is preferable to use polymeric particles copolymerized with a monomer having carboxyl group such as acrylic acid or methacrylic acid; for the purpose of immobilizing a specific binding substance and an enzyme.

The concrete kinds of the individual monomers are selected such that the resulting water-dispersible polymeric particles have a given glass transition temperature so as not to cause fusion or aggregation during use or storage when the water-dispersible polymeric particles immobilized with a first specific binding substance and an enzyme are used. The glass transition temperature of the water-dispersible polymeric particles is preferably 10°C or higher, particularly room temperature or higher.

The carrier used in the present invention may be colored. In a case where a colored carrier is used, when the concentration of an analyte is high, a determination based on the degree of coloration can be made even before carrying out an enzymatic reaction. The colored carrier used herein is not particularly limited as long as the coloration can be detected visually. The colored carrier includes, for instance, colloid particles made of metals such as gold, silver and copper; water-dispersible polymeric particles colored with pigments and dyes such as Sudan Blue, Sudan Red IV, Sudan III, Oil Orange, and Quinizarin Green, and the like. From the aspect of visual confirmation, it is preferable to use a gold colloid and water-dispersible polymeric particles colored with blue, red, green or orange pigments and dyes. More preferably, a blue- or red-colored latex is used as water-dispersible polymeric particles from the aspect of dispersion stability and easy adjustment of the detection sensitivity of the analyte.

The enzyme-labeled specific conjugate used in the present invention can be obtained by immobilizing a first specific binding substance and an enzyme onto a carrier such as water-dispersible polymeric particles. The particle size of the polymeric particles is preferably 3 µm or less, more preferably 2 µm or less, from the viewpoints of a uniform dispersion of the particles in an aqueous medium to prevent sedimentation thereof and of sufficient immobilization of the first specific binding substance and the enzyme, and the particle size is preferably 0.01 µm or more, more preferably 0.1 µm or more, from the viewpoint of facilitating the purification of the particles once immobilized.

The enzyme used as a label in the present invention may be any of those used conventionally in a solid phase immunoassay. Those exemplified concretely are peroxidases, β-D-galactosidase, alkaline phosphatases, glucose oxidases, luciferase, esterases, β-D-glucuronidase, and the like. Those preferred are peroxidases and alkaline phosphatases which are capable of achieving a stable detection with high sensitivity.

The first specific binding substance immobilized onto the water-dispersible polymeric particles is not particularly limited as long as it binds specifically to an analyte. Examples thereof are antigens, haptens, antibodies, oligonucleotides, effectors, receptors, enzymes, coenzymes, enzyme inhibitors and the like. One or more of the first specific binding substances may be appropriately selected from those known used in a usual detection method such as sandwich method depending on an analyte to be tested.

In the present invention, in particular, an immunochemical component such as an antigen, a hapten or an antibody is preferable. In other words, it is preferable to use, as an enzyme-labeled specific conjugate, a labeled conjugate comprising water-dispersible polymeric particles as a carrier and an immunochemical component selected from the group consisting of antigens, haptens and antibodies is immobilized onto the surface of the carrier, and an enzyme is immobilized thereon. The total immobilized amount of the immunochemical component and the enzyme is, as described below, preferably from 5 to 200 mg per 1 g of the water-dispersible polymeric particles on a dry basis. In the present invention, the enzyme-labeled specific conjugate may be simply referred herein as "labeled conjugate."

When the specific binding substance is an antigen or hapten, the antigen or hapten includes various microorganism antigens such as *Chlamydia trachomatis*, hemolytic streptococcus, *Bordetella pertussis*, *Helicobacter pylori, Leptospira, Treponema pallidum, Toxoplasma gondii,* and *Borrelia*; and mycoplasma lipid antigen, HA antigen, HBc antigen, HBe antigen, HBs antigen, HCV antigen, and HIV antigen; haptens derived from the antigens mentioned above, and the like.

When the specific binding substance is an antibody, the antibody can be a monoclonal antibody or a polyclonal antibody. Concrete examples thereof include anti-*E. coli* antibody, anti-*E. coli* 0157 antibody, anti-*Salmonella* antibody, anti-*Staphylococcus* antibody, anti-*Campylobacter* antibody, anti-*Clostridium perfringens* antibody, anti-*Vibrio parahaemolyticus* antibody, anti-Vero toxin antibody, anti-human transferrin antibody, anti-human albumin antibody, anti-human immunoglobulin antibody, anti-microglobulin antibody, anti-CRP antibody, anti-troponin antibody, anti-HCG antibody, anti-*Chlamydia trachomatis* antibody, anti-streptolysin-O antibody, anti-*Helicobacter pylori antibody,* anti-β-glucan antibody, anti-HBe antibody, anti-HBs antibody, anti-adenovirus antibody, anti-HIV antibody, anti-rotavirus antibody, anti-RF antibody, and the like.

When the specific binding substance is an oligonucleotide, the oligonucleotide includes oligonucleotides complementary to the nucleic acid components derived from various microorganisms, mycoplasma and various viruses exemplified above as antigens.

The water-dispersible polymeric particles used in the present invention may each have a functional group for the purpose of immobilizing a first specific binding substance and an enzyme onto the surface thereof, introducing a spacer, or increasing the stability in a state of water dispersion. Examples of the functional group are carboxyl group, hydroxyl group, glycidyl group, amino group,.,formyl group, carbamoyl group, isothiocyanate group, azidocarbonyl group, hydrazide group, acid anhydride group and the like, with a preference given to a case where carboxyl group is introduced. In order to prepare the water-dispersible polymeric particles each having the functional group mentioned above, a monomer having carboxyl group such as acrylic acid or methacrylic acid; a monomer having hydroxyl group such as 2-hydroxyethyl acrylate or 2-hydroxyethyl methacrylate; or a monomer having glycidyl group such as glycidyl methacrylate is used as a monomer component, and subjected to an emulsion copolymerization, if necessary, with other copolymerizable monomers, so that the water-dispersible polymeric particles each having carboxyl group, hydroxyl group or glycidyl group can be obtained. Alternatively, a certain amount of the monomer component is polymerized, and thereafter the functional group can be introduced to the resulting water-dispersible polymeric particles.

The first specific binding substance and/or the enzyme are immobilized onto the carrier used in the present invention by means of a hydrophobic bond (physical adsorption), an ionic bond, a covalent bond, and the like. From the viewpoint of stability, it is preferable to immobilize firmly by means of a covalent bond.

Also in the present invention, when linking the water-dispersible polymeric particles with the first specific binding substance and/or with the enzyme via a covalent bond, if necessary, a spacer group may be inserted in order to enhance the freedom of the first specific binding substance and/or the enzyme on the polymeric particles. The spacer group may first be linked to the water-dispersible polymeric particles and subsequently linked to the first specific binding substance and/or the enzyme. Alternatively, the spacer group may first be linked to the first specific binding substance and/or the enzyme, and subsequently linked to the water-dispersible polymeric particles. Furthermore, the spacer group may previously be linked to each of the water-dispersible polymeric particles, the first specific binding substance and the enzyme, and each spacer group-linked compound is then linked to the remaining parties.

The compound which can be used as a spacer group is an organic compound having at least bifunctional group, and especially preferably an organic compound with a carbon chain of 1 to 12 carbon atoms having bifunctional group, but a polymer having a multifunctional group is by no means excluded. Concrete examples of the compound serving as a spacer group are diamines, such as hexamethylenediamine, dodecamethylenediamine and xylylenediamine; aminoalkylcarboxylic acids, such as glycine, β-aminopropionic acid, γ-aminobutyric acid, ε-aminocaproic acid and ε-aminocaprylic acid; amino acids such as lysine, glutamic acid, β-alanine, arginine and glycylglycine and the like, without being limited thereto.

The method for linking covalently a first specific binding substance and/or an enzyme to water-dispersible polymeric particles directly or via a spacer group is not particularly limited, and may be any of those known per se. For example, a preferable method includes a method using a water-soluble carbodiimide as a binding reagent. In a case where an aminoalkylcarboxylic acid is used as a spacer group, for instance, the aminoalkylcarboxylic acid is linked to water-dispersible polymeric particles using a water-soluble carbodiimide, and then to the aminoalkylcarboxylic acid which has been linked to the polymeric particles is linked covalently a first specific binding substance and/or an enzyme using the water-soluble carbodiimide in the same manner as above.

The water-soluble carbodiimide used in the method described above includes 1-ethyl-3-(3-dimethylaminopropyl)carbodiimide hydrochloride, 1-cyclohexyl-3-(2-morpholinoethyl)carbodiimide-metho-p-toluenesulfonate, and the like. In order to link covalently, directly without a spacer group or via a spacer group, the first specific binding substance and/or the enzyme to the water-dispersible polymeric particles using a water-soluble carbodiimide mentioned above, conventionally known methods and conditions can be employed. In the present invention, the specific binding substance and the enzyme are linked to a carrier by immobilizing them simultaneously or separately.

In addition to the case where a single first specific binding substance is immobilized, a mixture of several first binding substances each of which binds specifically to one of several analytes can be immobilized, if necessary.

Thus, the present invention may also be directed to the method for detection of a plurality of different analytes, in addition to the method for detection of a single analyte. In this case, there are two embodiments for the enzyme-labeled specific conjugate: 1) an embodiment where the enzyme-labeled specific conjugate results from linking via a carrier, an enzyme and a single first specific binding substance capable of specifically binding to a single analyte; and 2) an embodiment where the enzyme-labeled specific conjugate results from linking, via a carrier, an enzyme and a plurality of first specific binding substances each of which is capable of specifically binding to one of a plurality of analytes. The Embodiment 1) uses a plurality of different enzyme-labeled specific conjugates each of which is capable of specifically binding to one of the plurality of different analytes, and a plurality of complexes are formed in Step (1) of the method for detection of the present invention. On the other hand, in the case of the Embodiment 2), there can be formed a complex of one enzyme-labeled specific conjugate and the plurality of different analytes.

The total immobilized amount of the first specific binding substance and the enzyme contained in the enzyme-labeled specific conjugate obtained as described above is preferably 5 to 200 mg per 1 g of the carrier on a dry basis, and the amount can vary appropriately within the above range depending on the kinds of specific binding substances and enzymes used as well as the extent of activity. For example, when the carrier is water-dispersible polymeric particles, the total immobilized amount is preferably 200 mg or less, more preferably 150 mg or less, per 1 g of the water-dispersible polymeric particles on a dry basis, in consideration of the surface area of the particles, and the total immobilized amount is preferably 5 mg or more, and more preferably 10 mg or more, from the viewpoints of rapid detection and higher sensitivity and reproducibility.

Here, the term "on a dry basis" of the water-dispersible polymeric particles refers to a weight of a given amount of the water-dispersible polymeric particles after being dried at 120°C for 2 hours.

When an enzyme-labeled specific conjugate is used expecting to achieve a further rapid and highly sensitive immunoassay, an enzyme to be immobilized to a carrier can be appropriately varied depending on the kinds thereof. For the purpose of rapid detection and higher sensitivity and reproducibility, it is preferable that the amount of the enzyme to be immobilized is 1.0 mg or more, per 1 g of the water-dispersible polymeric particles on a dry basis. For the same reasons as above, it is preferable that the amount of the enzyme to be immobilized is 100 mg or less, per 1 g of the water-dispersible polymeric particles on a dry basis.

The immobilized amount of the enzyme is preferably 0.6 mol or more, per one mol of the first specific binding substance, and from the viewpoints of the surface area of the water-dispersible polymeric particles, and of reducing the background level, it is preferably 60 mol or less, per one mol of the first specific binding substance.

In the present invention, the amount of an enzyme to be immobilized can also be expressed as its activity. It is a matter of course that the enzyme activity may vary depending on various conditions, such as the kinds of the enzyme to be immobilized, the substrate therefor and the temperature. When the enzyme to be immobilized onto a carrier is a peroxidase, the activity is determined by the following method. In 3.3 ml of 0.1 M citrate buffer (pH 4.5) containing 0.2 mg/ml of a substrate TMB (3,3',5,5'-tetramethylbenzidine) and 0.01 % of hydrogen peroxide is mixed 100 µl of 0.0125% suspension of an enzyme-labeled specific conjugate. The resulting mixture is reacted at 25°C during which the absorbances at 580 nm are determined with the passage of time, wherein the enzymatic activity which increases the absorbance by 1 (OD) in 1 minute is counted as 1 U (unit).

In the case of a peroxidase, it is desired that the enzyme is immobilized such that the enzyme-labeled specific conjugate has an enzymatic activity of 5,000 to 300,000 U, preferably 10,000 to 300,000 U, more preferably 50,000 to 300,000 U, per 1 g of the water-dispersible polymeric particles on a dry basis.

When the enzymatic activity is in the above ranges, it would facilitate the detection of an analyte rapidly and reduce background level, thereby giving high specificity.

The immobilized amounts of the first specific binding substance and the enzyme are determined by calculating as an amount of proteins based on the measurement using Lowry's method. When the specific binding substance is an oligonucleotide, the calculation is based on the free oligonucleotide after immobilization, as determined by measuring the absorbances of the supernatant after immobilization at 260 nm.

After linking the first specific binding substance and the enzyme to the water-dispersible polymeric particles as described above, i.e., after preparing an enzyme-labeled specific conjugate, the enzyme-labeled specific conjugate according to the present invention can be isolated and purified by a conventional usual separation process such as membrane separation, filtration and centrifugation. The enzyme-labeled specific conjugate according to the present invention thus obtained may be stored by impregnating it in water or lyophilizing it.

Next, the method for detection according to the present invention will be explained by following each of the steps.

### A. Embodiment for Detecting One Analyte

### (1) Step of forming a complex of an enzyme-labeled specific conjugate and an analyte to wick through a water-absorbent substrate

The complex of an enzyme-labeled specific conjugate and an analyte may be previously formed before wicking through a water-absorbent substrate, or alternatively, as described below, a complex may be formed on a water-absorbent substrate by bringing a test solution containing an analyte into contact with an enzyme-labeled specific conjugate contained in a dried reagent region in the course of the wicking through the water-absorbent substrate. Further alternatively, an analyte loaded onto a water-absorbent substrate may be brought into contact with a dispersion of an enzyme-labeled specific conjugate, whereby forming a complex on the water-absorbent substrate.

When a complex is previously formed, a dispersion of an enzyme-labeled specific conjugate is mixed with a test solution containing an analyte, or an analyte is admixed with a dispersion of an enzyme-labeled specific conjugate, or an enzyme-labeled specific conjugate is added and dispersed in a test solution containing an analyte, and thereafter the resulting mixture may be wicked through a water-absorbent substrate.

As a method for wicking, a solution of a complex obtained as described above is loaded onto one end of a water-absorbent substrate, which is allowed to wick as a result of a capillary phenomenon. In order to facilitate the loading of the solution onto a water-absorbent substrate, a water-absorbable pad may be provided at the site of the loading. The water-absorbent pad may be provided at the end opposite to the site of the loading of the solution to absorb the liquid wicked through the water absorbent substrate, whereby allowing rapid progress of the wicking.

The water-absorbent substrate used in the present invention is not particularly limited, as long as it can absorb a test solution containing an analyte, such as plasma, blood, urine, feces and saliva, as well as diluted solutions thereof prepared by diluting them with buffers. In the present invention, a water-absorbent substrate which can securely have a sufficient time period to carry out the reaction of an analyte in a test solution with a first specific binding substance or with a second specific binding substance in a capture region in Step (2) described below is used. When a water-absorbent substrate has good absorbability, a time period for the test solution to reach the capture region is sufficiently short enough to perform rapid determination and long enough to perform an accurate determination. Preferred concrete examples are nonwoven fabrics, filter papers, glass fiber fabrics, glass filters, nitrocelluloses, porous substrates, and the like. Each of these substrates has a proper water absorption rate and enables an excellent visual confirmability owing to color development upon aggregation of the carrier when a colored carrier is used.

In consideration of the aspects described above, the degree of water absorbability of the water-absorbent substrate according to the present invention is preferably such that the water absorption distance is about 0.5 to about 5 cm at 1 minute after immersing in water one end of the water-absorbent substrate in the form of a 5 mm-width strip.

Also, in order to adjust the water absorbability of the substrate, the surface of the substrate may be coated or impregnated with a hydrophilic polymer. Further, a continuous substrate obtained by binding the edges of substrates made of the same material or of different materials for the water-absorbent substrate to each other by any suitable binding means may also be used.

In the present invention, the shape of the water-absorbent substrate is not particularly limited as long as the water-absorbent substrate has a shape capable of wicking the test solution. The shape, for instance, is preferably a rectangular sheet (strip) or a rod shape.

In the present invention, as another embodiment, an enzyme-labeled specific conjugate may previously be further contained in the water-absorbent substrate, so that the enzyme-labeled specific conjugate is arranged such that the enzyme-labeled specific conjugate can leave from and wick through the water-absorbent substrate when contacted with water. In this embodiment, there is no need of subsequent separate addition of an enzyme-labeled specific conjugate. The method in which the enzyme-labeled specific conjugate is contained includes, for example, a process comprising applying a solution of an enzyme-labeled specific conjugate onto a water-absorbent substrate, and subsequently drying under appropriate conditions. As an example of drying process, after the solution is applied onto a water-absorbent substrate, the substrate is air-dried by feeding air at 20° to 60°C, or by lyophilization. As an alternative method, an enzyme-labeled specific conjugate is dispersed in a solution of a water-soluble polymer or saccharose, and the resulting dispersion is applied onto a water-absorbent substrate, and then dried. This method is advantageous in preparing a test strip which is a test reagent for the present invention. When contacted with water, the water-soluble polymer or saccharose is readily solubilized in water, so that the enzyme-labeled specific conjugate quickly leaves from and wick through the substrate, and reacted with an analyte to form a complex. In addition, since a solution having an appropriate viscosity can be obtained by adjusting the concentration of the water-soluble polymeric or saccharose, the enzyme-labeled specific conjugate not only can readily be contained at a given region of the water-absorbent substrate but also is difficult to undergo aggregation or denaturation when drying. Further, the enzyme-labeled specific conjugate is less likely to leave from the water-absorbent substrate after drying.

By using the water-absorbent substrate containing the enzyme-labeled specific conjugate as described above, a complex can be formed by bringing a test solution containing an analyte in contact with the enzyme-labeled specific conjugate in the course of the wicking through the water-absorbent substrate. In the present invention, the region at which an enzyme-labeled specific conjugate is contained in the water-absorbent substrate as described above is referred to as a dried reagent region.

Preferred examples of the water-soluble polymer described above are polyvinyl pyrrolidones, polyvinyl alcohols, polyethylene glycols, cellulose ethers (such as methyl cellulose, ethyl cellulose, carboxymethyl cellulose, carboxyethyl cellulose, oxyethyl cellulose and cyanoethyl cellulose), gelatin, and the like.

In order to wick the enzyme-labeled specific conjugate through the substrate in this embodiment, a test solution is loaded on a sample receiver positioned upstream of the dried reagent region or at the position same as the dried reagent region. When an analyte is applied directly onto the sample receiver, a solvent capable of solubilizing the analyte is loaded on the sample receiver. The solution wicks through a water-absorbent substrate owing to a capillary phenomenon. In this embodiment, a water-absorbent pad may be arranged inside the sample receiver, or it may be arranged at the downstream end of the sample receiver.

### (2) Step of capturing the complex of Step (1) in a capture region in which a second specific binding substance capable of specifically binding to an analyte is immobilized onto the water-absorbent substrate

Step (2) requires a capture region in which a second specific binding substance capable of specifically binding to an analyte is immobilized onto the water-absorbent substrate used in Step (1).

The second specific binding substance may be the same ones as those described above for the first specific binding substance, which may be selected appropriately depending on the first specific binding substance.

When the first specific binding substance is an antibody, the same antibody may be used for the second specific binding substance, or an antibody recognizing another epitope may also be used therefor. When the first specific binding substance is an antigen or a hapten, a second specific binding substance may be the same antigen or hapten, or it may be an antigen, an antibody or a hapten, which is a binding substance different from the first specific binding substance and being specifically bound to an analyte. When the first specific binding substance is an oligonucleotide, an oligonucleotide complementary to a nucleotide sequence of another portion of an analyte different from the sequence complementary to the oligonucleotide of the first specific binding substance can be used.

The method for immobilizing a second specific binding substance onto a water-absorbent substrate is not particularly limited, and it is preferably carried out by means of conventionally known physical adsorption or covalent bond. Alternatively, a solution containing a second specific binding substance and a hydrophilic polymer is applied onto a water-absorbent substrate, and the resulting substrate is subsequently immersed in a solidifying solvent capable of solidifying the hydrophilic polymer, whereby forming a capture region. The hydrophilic polymer includes hydroxypropyl methyl cellulose, polyvinyl alcohols, hydroxyethyl cellulose, and the like. The solidifying solvent described above includes acetone, ethanol, methanol, ethers, and the like.

The water-absorbent substrate after immobilization is preferably used after treatment with a solution containing proteins, surfactants and saccharides. Examples of the proteins to be used herein are calf serum albumin, casein, gelatin, skim milk, and the like. Examples of the surfactants are polyoxyethylene(10) octyl phenyl ethers, polyoxyethylene sorbitan monolaurates, polyoxyethylene alkyl allyl ether phosphates, polyoxyethylene alkyl ether phosphates, polyoxyethylene alkyl phenyl ethers, and the like. Examples of the saccharides are glucose, trehalose, saccharose, and the like. The treatment can be simply carried out by dissolving each component described above in water, and immersing a water-absorbent substrate after immobilization in the resulting solution. The proteins mentioned above serve to prevent the adsorption of the unnecessary proteins onto the surface of the water-absorbent substrate, and the surfactant serves to facilitate the wicking, and the saccharides serve to increase the stability of the second specific binding substance applied on the surface of the water-absorbent substrate.

The protein content in a treatment solution is preferably 0.1 to 10% by weight, within which range high reactivity is effectively maintained in the capture region. The surfactant content is preferably 0.01 to 1% by weight, within which range the wicking is effectively carried out and high reactivity is maintained in the capture region. The saccharide content is preferably 0.1 to 10 % by weight, within which range the solution tends to efficiently and effectively wick through the water-absorbent substrate.

In the capture region thus formed, an immobilized second specific binding substance binds to an analyte moiety of the complex formed in Step (1), whereby capturing the complex.

### (3) Step of detecting the captured complex

### i) Method for Detection Using Enzymatic Reaction

When the enzyme-labeled specific conjugate used in the present invention is used in various immunoassays, conventional color development substrates and detection methods which are known per se are selected depending upon the kinds of an enzyme immobilized, and an analyte is detected and quantified on the basis of the degree of color development and the change in the activity. The substrate used in this Step for an alkaline phosphatase includes, for example, may be p-nitrophenyl phosphate, 5-bromo-4-chloro-3-phosphate/nitro blue tetrazolium, first red/naphthol, AS-TR phosphate, and the like. For a peroxidase, those exemplified are combinations of hydrogen peroxide with one compound selected from 2,2'-azino-bis(3-ethylbenzthiazoline)-6-sulfonic acid, o-phenylenediamine, 3,3',5,5'-tetramethylbenzidine, o-dianisidine, 5-aminosalicylic acid, 3,3'-diaminobenzidine, 3-amino-9-ethylcarbazole, 4-chloro-1-naphthol, and the like.

### ii) Method for Detection Using Colored Carrier

When a large amount of an analyte is examined, a complex containing a colored carrier captured in a capture region can be detected visually.

### B. Embodiment for Detecting Plurality of Different Analytes

### (1) Step of forming a complex of an enzyme-labeled specific conjugate and a plurality of different analytes, the enzyme-labeled specific conjugate of which results from linking via a carrier, an enzyme and a plurality of first specific binding substances each of which is capable of specifically binding to one of the analytes to wick through a water-absorbent substrate

The complex of an enzyme-labeled specific conjugate and an analyte may previously be formed before wicking through a water-absorbent substrate, or alternatively, as described below, a complex may be formed on a water-absorbent substrate by bringing a test solution containing a plurality of different analytes into contact with an enzyme-labeled specific conjugate contained in a dried reagent region in the course of the wicking through the water-absorbent substrate. Further alternatively, a plurality of different analytes loaded onto a water-absorbent substrate may be brought into contact with a dispersion of an enzyme-labeled specific conjugate, whereby forming a complex on the water-absorbent substrate. As described above, in the present invention, there can be used an enzyme-labeled specific conjugate resulting from linking via one carrier, an enzyme and a single first specific binding substance capable of specifically binding to a single analyte; or an enzyme-labeled specific conjugate resulting from linking, via one carrier, an enzyme and a plurality of first specific binding substances each of which is capable of specifically binding to one of the plurality of different analytes. Therefore, in the former case, a plurality of complexes are formed depending on the number of analytes, and in the latter case, a complex resulting from binding a plurality of analytes to a single enzyme-labeled specific conjugate is formed.

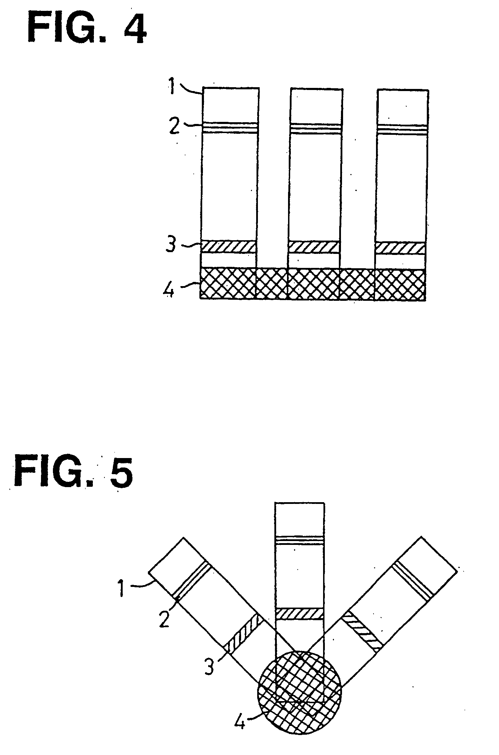

In this Embodiment B, the same can be said as described in Embodiment A above unless otherwise specified. For instance, the method for previously forming a complex, the method for wicking, and the water-absorbent substrate are the same as those given in Embodiment A described above. Also the same can be said as in Embodiment A in that the enzyme-labeled specific conjugate may previously be contained in a water-absorbent substrate, so that the enzyme-labeled specific conjugate can leave from and wick through the water-absorbent substrate when contacted with water, and the region where an enzyme-labeled specific conjugate is contained in the water-absorbent substrate is referred to as a dried reagent region. Also in the present invention, when two or more enzyme-labeled specific conjugates are used, they may be mixed and applied to a water-absorbent substrate to form a single dried reagent region (see Figure 2), or each of the enzyme-labeled specific conjugates is applied individually to form a plurality of dried reagent regions (see Figures 3, 4 and 5). In the latter case, a plurality of dried reagent regions may be formed individually on a single water-absorbent substrate (see Figure 3), or a plurality of dried reagent regions may be formed individually on each of the plurality of water-absorbent substrates (see Figures 4 and 5). The method for wicking when a dried reagent region is formed is also the same as that described in Embodiment A.

### (2) Step of capturing a complex in a plurality of capture regions onto the water-absorbent substrate in which second specific binding substances each of which is capable of specifically binding to one of the plurality of different analytes are immobilized thereon

Step (2) requires a plurality of capture regions onto the water-absorbent substrate used in Step (1) in which second specific binding substances each of which is capable of specifically binding to one of the analytes are immobilized thereon. In the present invention, the term "a plurality of capture regions" referred herein means a region in which a plurality of second specific binding substances each of which is capable of specifically binding to one of the plurality of analytes are immobilized. Here, each region in which each of the second specific binding substances is immobilized is separately formed on a single water-absorbent substrate to constitute a plurality of capture regions (See Figures 2 and 3), or alternatively, a plurality of second specific binding substances may individually be immobilized onto each of a plurality of water-absorbent substrates by using the plurality of water-absorbent substrates and a sample receiver may be shared therebetween (See Figures 4 and 5).

Also in this Step, the description of the second specific binding substance, the method for immobilizing a second specific binding substance onto a water-absorbent substrate, and the post-immobilization treatment of the water-absorbent substrate are the same as those given in Embodiment A described above.

### (3) Step of detecting the captured complex

This Step is also carried out by a method for detection using an enzymatic reaction or a method for detection using a colored carrier in the same manner as in Embodiment A.

Next, a test strip used as a test reagent according to the present invention will be described below.

### A. Embodiment for Detecting One Analyte

The test strip according to the present invention is suitably used in a method for detection of the present invention, in which one of the features thereof resides in the use of an enzyme-labeled specific conjugate resulting from linking, via a carrier, an enzyme and a first specific binding substance capable of specifically binding to an analyte. Concretely, as shown in Figure 1, the test strip comprises a water-absorbent substrate 1; a capture region 2 in which a second specific binding substance capable of specifically binding to an analyte is immobilized onto the water-absorbent substrate 1; a dried reagent region 3 comprising an enzyme-labeled specific conjugate resulting from linking, via a carrier, an enzyme and a first specific binding substance capable of specifically binding to the analyte described above in such a manner that the enzyme-labeled specific conjugate leaves from and wicks through the substrate when contacted with water; and a sample receiver 4 positioned upstream of the dried reagent region or at the same position as the dried reagent region (Figure 1 illustrates an embodiment where a sample receiver is arranged upstream of a dried reagent region).

The water-absorbent substrate, the capture region and the dried reagent region used herein are similar to those described above with regard to the method for detection according to the present invention. In the capture region, the second specific binding substance preferably in an amount of 0.005 to 5 mg/cm² is applied to the water-absorbent substrate in order to capture a complex migrating and wicking as a result of absorption of the test solution. When the amount of the second specific binding substance is within the above range, the complex capturing efficiency and the detection sensitivity can be well maintained, thus having economic advantages. Also, the amount of the enzyme-labeled specific conjugate to be contained in a dried reagent region is, for instance, preferably 0.5 to 50 µg for a water-absorbent substrate in the form of a strip of which dimensions are such that a width is 6 mm, and a length is 6 cm.

In addition, in the present invention, the capture region is arranged downstream of the dried reagent region, and the distance between the capture region and the dried reagent region is 0.5 cm or longer, preferably 1 cm or longer, from the viewpoints of better uniformity and sensitivity in color development in the capture region, and the distance is 6 cm or shorter, preferably 4 cm or shorter, from the viewpoints of satisfactory ability of the test solution to reach the capture region, the sensitivity in color development, and the testing time period.

The sample receiver is not particularly limited as long as it is positioned upstream of the dried reagent region or at the same position as the dried reagent region described above to which a sample can be loaded. The sample receiver may be a water-absorbent substrate itself, or a water-absorbent pad capable of retaining an aqueous solution temporarily. The water-absorbent pad includes, for example, nonwoven fabrics made from polyesters, rayons, polypropylenes, celluloses, pulps, and the like. When a sample receiver is arranged at the same position as the dried reagent region, the dried reagent region is prepared, and thereafter a water absorbent pad constituting a sample receiver may be laminated thereon, or a dried reagent region may be arranged inside the sample receiver.

The dimensions of the sample receiver are preferably such that each of length and width is 3 to 10 mm, and its thickness is 0.1 to 5 mm.

The sample receiver described above is arranged at one end, or in its proximity, of the water-absorbent substrate having the capture region and the dried reagent region. The sample receiver is arranged by placing it on a water-absorbent substrate, and then pressing it physically with a test strip case, or pasting with a glue.

In order to carry out the detection with the test strip according to the present invention, a test solution to be analyzed is allowed to be absorbed from a sample receiver positioned upstream of the dried reagent region. The test solution wicks and migrates through a water-absorbent substrate, and arrives at a dried reagent region, where the enzyme-labeled specific conjugate leaves from the substrate. At this time, the analyte contained in the test solution is bound to the enzyme-labeled specific conjugate, whereby forming a complex. When the sample receiver is arranged at the same position as the dried reagent region, a complex is formed at this position by addition and absorption of the test solution.

Subsequently, a complex thus formed wicks and migrates through the water-absorbent substrate along with the migration of the test solution and arrives at the capture region. In the capture region, the complex which has migrated is further bound with a second specific conjugate immobilized at the capture region to form a new complex of an enzyme-labeled specific conjugate of the enzyme-labeled specific conjugate (enzyme-carrier-first specific binding substance)-analyte-second specific binding substance, which is captured on the capture region. When the concentration of an analyte is high and the carrier is colored, the complex can be concentrated and aggregated in one location by capturing and immobilizing the complex at the capture region as described above, to clearly give color development, whereby the presence of the analyte can be visually confirmed.

When the concentration of an analyte is low, the detection at a higher sensitivity can be accomplished by adding to the capture region a substrate for an immobilized enzyme. The substrate used herein can be any one capable of color development by reaction with an immobilized enzyme. In the case of an alkali phosphatase, those exemplified are p-nitrophenyl phosphate, 5-bromo-4-chloro-3-phosphate/nitro blue tetrazolium, first red/naphthol, AS-TR phosphate and the like. In the case of a peroxidase, those exemplified are combinations of hydrogen peroxide with one compound selected from 2,2'-azino-bis(3-ethylbenzthiazoline)-6-sulfonic acid, o-phenylenediamine, 3,3',5,5'-tetramethylbenzidine, o-dianisidine, 5-aminosalicylic acid, 3,3'-diaminobenzidine, 3-amino-9-ethylcarbazole, 4-chloro-1-naphthol, and the like.

### B. Embodiment of Detecting Plurality of Different Analytes

One feature of this embodiment resides in the use of a water-absorbent substrate comprising a plurality of capture regions in which a plurality of second specific binding substances each of which is capable of specifically binding to one of a plurality of different analytes are immobilized, and a dried reagent region comprising an enzyme-labeled specific conjugate resulting from linking via a carrier, an enzyme and a first specific binding substance capable of binding specifically to the analyte. Concretely, as shown in Figures 2 to 5, the test strip comprises a water-absorbent substrate 1; a capture region 2 in which a plurality of second specific binding substances each of which is capable of specifically binding to one of the plurality of different analytes are immobilized onto the water-absorbent substrate 1; a dried reagent region 3 comprising an enzyme-labeled specific conjugate resulting from linking, via a carrier, an enzyme and a first specific binding substance capable of specifically binding to the analyte described above in such a manner that a complex or complexes are formed between the enzyme-labeled specific conjugate and the plurality of different analytes, and the enzyme-labeled specific conjugate leaves from the substrate when contacted with water; and a sample receiver 4 positioned upstream of the dried reagent region or at the same position as the dried reagent region (Each of Figures 2 to 5 illustrates an embodiment where a sample receiver is positioned upstream of a dried reagent region). With regard to the dried reagent region 3, each of Figures 2, 4 and 5 illustrates an embodiment having a single dried reagent region, and Figure 3 illustrates an embodiment having a plurality of dried reagent regions. With regard to the sample receiver 4, each of Figures 4 and 5 illustrates an embodiment having a shared sample receiver. Incidentally, as in cases illustrated by Figures 2 and 3, each test strip represents the test strip according to the present invention, while as in cases illustrated by Figures 4 and 5, a cluster of a plurality of strips as a whole represents the test strip according to the present invention. The shape of the test strip of the present invention is not particularly limited, and it may, for example, be in the form of a rectangular strip as shown in Figures 2 to 5.

The descriptions of the capture region, the dried reagent region and the sample receiver used in this embodiment, and the method for detection using the test strip described above are the same as those given in Embodiment A.

Also in this Embodiment, instead of providing an enzyme-labeled specific conjugate in a test strip as a dried reagent region as described above, a complex of an enzyme-labeled specific conjugate and an analyte may previously be formed, and then loaded on a water-absorbent substrate, in which the enzyme-labeled specific conjugate wicks therethrough, or alternatively an analyte and an enzyme-labeled specific conjugate are sequentially added dropwise to a sample receiver, and then allowed to form a complex inside of the sample receiver or on the water-absorbent substrate, the enzyme-labeled specific conjugate wicks therethrough. In this case, the test strip used has no dried reagent region, but comprises a single capture region (Embodiment A), or a plurality of capture regions (Embodiment B), each being immobilized onto a water-absorbent substrate, and a sample receiver positioned upstream thereof.

Therefore, according to the present invention, there can be provided:
[1] a test kit of an analyte comprising:
   (1) a test strip comprising a capture region arranged on a water-absorbent substrate, comprising a second specific binding substance capable of specifically binding to the analyte, and a sample receiver arranged at a position upstream of the capture region; and
   (2) an enzyme-labeled specific conjugate resulting from linking via a carrier, an enzyme and a first specific binding substance capable of specifically binding to the analyte; or
[2] A test kit of a plurality of analytes comprising:
   (1) a test strip comprising a plurality of capture regions arranged on a water-absorbent substrate, each comprising a second specific binding substance capable of specifically binding to one of the plurality of different analytes, and a sample receiver arranged at a position upstream of the capture regions; and
   (2) an enzyme-labeled specific conjugate resulting from linking via a carrier, an enzyme and a first specific binding substance capable of specifically binding to the analyte.

The test strips used in the above kits are similar to those of the test strip of the present invention except for not providing a dried reagent region.

### EXAMPLES

### Example 1: Preparation of Enzyme-Labeled Specific Conjugate (Enzyme-Antibody Immobilized Carrier)

### 1) Preparation of Water-Dispersible Polymeric Particle Suspension

A liquid mixture comprising 50 g of styrene, 0.5 g of acrylic acid, 0.2 g of triethylene glycol methacrylate, and 440 g of distilled water was kept at a temperature of 75°C under a nitrogen gas atmosphere. With stirring, to the resulting liquid mixture was added an aqueous solution prepared by dissolving 0.25 g of potassium persulfate as a polymerization initiator in 10 g of distilled water, and the components were polymerized for 10 hours. As a result, carboxylated polystyrene latex particles (average particle size: 0.2 µm) were obtained as carboxylated water-dispersible polymeric particles. The resulting carboxylated polystyrene latex particles were dispersed in 0.01 M-borate buffer at pH 8.2 so as to have a concentration of the solid components of 5% by weight.

### 2) Immobilization

### i) Immobilization of Specific Binding Substance

In this Example, as a specific binding substance, an antibody was immobilized on the latex particles prepared in Item 1) above in the following manner.

To 3 ml of a dispersion of the latex particles were added 0.5 ml of a water-soluble carbodiimide [manufactured by DOJINDO LABORATORIES; 1-ethyl-3-(3-dimethylaminopropyl)carbodiimide hydrochloride at 10 mg/ml in 0.01 M-borate buffer at pH 8.2], and 2 ml of a goat anti-*E. coli* O157:H7 polyclonal antibody (manufactured by Kirkegaard & Perry Laboratories Inc. at 1 mg/ml in 0.01 M-borate buffer at pH 8.2), and the resulting mixture was reacted at 10°C for 3 hours. Thereafter, washing-by-centrifugation was carried out with 0.01 M-borate buffer at pH 8.2 as a washing liquid, and the concentration of the solid components was adjusted to be 5% by weight with the same buffer.

### ii) Immobilization of Enzyme

Next, to 3 ml of the suspension of the antibody-immobilized latex particles prepared above were added 1 ml of a water-soluble carbodiimide [manufactured by DOJINDO LABORATORIES; 1-ethyl-3-(3-dimethylaminopropyl)carbodiimide hydrochloride at 10 mg/ml in 0.01 M-borate buffer at pH 8.2] and 2 ml of a horseradish-derived peroxidase (hereinafter simply referred to as "HRP," manufactured by Wako Pure Chemical Industries, Ltd. at 1.2 mg/ml in 0.01 M-borate buffer at pH 8.2), and the resulting mixture was reacted at 10°C for 3 hours. Thereafter, washing-by-centrifugation was carried out with 0.01 M-borate buffer at pH 8.2 as a washing liquid, and the concentration of the solid components was adjusted to be 2% by weight with the same buffer, to prepare latex particles immobilized with the goat anti-*E. coli* O157:H7 polyclonal antibody and the HRP.

### Example 2

Entirely the same procedures as in Example 1 were carried out except for using an HRP prepared to a concentration of 12 mg/ml in place of the HRP prepared to a concentration of 1.2 mg/ml when immobilizing the enzyme, to prepare latex particles immobilized with the goat anti-*E. coli* O157:H7 polyclonal antibody and the HRP.

### Example 3

Entirely the same procedures as in Example 1 were carried out except for using an HRP prepared to a concentration of 60 mg/ml in place of the HRP prepared to a concentration of 1.2 mg/ml, to prepare latex particles immobilized with the goat anti-*E. coli* O157:H7 polyclonal antibody and the HRP.

### Experimental Example 1: Determination of Amount of Antibody and Enzyme Immobilized

With respect to each of the latex particles immobilized with the goat anti-*E. coli* O157:H7 polyclonal antibody and the HRP prepared in Examples 1 to 3, the supernatant after immobilization of each of the antibody and the enzyme was subjected to quantification of protein, and each of the immobilized amount per 1 g of the latex particles on a dry basis was calculated therefrom.

The quantification of protein was carried out as follows.

### [Protein Quantification-Dye Binding Method (Bradford Method)]

As the quantification for amounts of immobilized antibodies and enzymes, a dye binding method with Coomassie Brilliant Blue G-250 was employed. The actual measurement was made with a commercially available kit "Coomassie Protein Assay Reagent" (manufactured by PIERCE). The measurement method was carried out according to the protocols attached.

The calibration curve was prepared by measuring absorbance at 595 nm after color development of the antibody solution or enzyme solution of a known concentration with the above reagent. The supernatant obtained after immobilization of each of antibodies and enzymes which was appropriately diluted was color-developed with the above reagent, and the absorbances at 595 nm were then measured to calculate the concentrations of the antibody and the enzyme from the calibration curve.

Measurements were taken at room temperature (25°C). The reaction period of time is not particularly limited, and the absorbance measurement was taken within 90 minutes after color development.

As a result, the amount of the antibody (molecular weight: about 160,000) immobilized for each of Examples was 11.1 mg, and the amount of the enzyme (molecular weight: about 40,000) immobilized for Example 1 was 1.8 mg, that for Example 2 was 17.3 mg, and that for Example 3 was 35.1 mg.

In addition, the number of enzymes per one molecule of the antibody was calculated from the above results, and it was found to be 0.6 molecules in Example 1, 6.2 molecules in Example 2, and 12.6 molecules in Example 3.

### Experimental Example 2: Determination of Enzyme Activity of Latex Particles Immobilized with Antibody and Enzyme

With respect to each of the latex particles immobilized with the goat anti-*E. coli* O157:H7 polyclonal antibody and the HRP prepared in Examples 1 to 3, the enzyme activity thereof was measured. In this experiment, TMB (3,3',5,5'-tetramethylbenzidine) membrane Peroxidase Substrate System (manufactured by Kirkegaard & Perry Laboratories Inc., pH 4.5) was used as a substrate solution. In a cuvette were mixed 0.1 ml of the dispersion of the enzyme-antibody immobilized latex particles diluted to 0.0125% by weight and 3.3 ml of the above substrate solution, and the mixture was reacted at 25°C. Thereafter, a 1 minute absorbance increase at 580 nm was measured by spectrophotometer. The activity by which the absorbance can be raised by 1 in 1 minute is defined as 1 U (unit), and expressed in terms of the activity per 1 g of the latex particles on a dry basis.

As a result, the activity per 1 g of the latex particles on a dry basis was 10,160 U in Example 1, 85,230 U in Example 2, and 267,180 U in Example 3.

### Comparative Example 1: Non Enzyme-Immobilized Carrier

In a dispersion of blue colored-carboxylated polystyrene latex particles (concentration of solid components: 5% by weight; average particle size: 0.1 µm; 0.01 M-borate buffer at pH 8) were added 0.5 ml of a water-soluble carbodiimide [manufactured by DOJINDO LABORATORIES; 1-ethyl-3-(3-dimethylaminopropyl)-carbodiimide hydrochloride at 10 mg/ml in 0.01 M-borate buffer at pH 8], and 2 ml of a goat anti-*E. coli* O157:H7 polyclonal antibody (manufactured by Kirkegaard & Perry Laboratories Inc. at 1 mg/ml in 0.01 M-borate buffer at pH 8), and the resulting mixture was reacted at 10°C for 3 hours. Thereafter, washing-by-centrifugation was carried out with 0.01 M-borate buffer at pH 8 as a washing liquid, to prepare blue colored latex particles-labeled anti-*E. coli* O157:H7 antibody (concentration of the solid components: 2% by weight).

### Example 4: Use of Test Strips for Immunochromatography

### 1) Preparation of Test Strips

At a position 30 mm from one end of the nitrocellulose membrane (pore size: 8 µm, dimensions: 6 mm × 60 mm) was applied with a dispenser in a line (capture region) 1.5 µl of a goat anti-*E. coli* O157:H7 polyclonal antibody (at 1 mg/ml in 0.1 M-phosphate buffer at pH 7.4).

The resulting membrane was immersed in an aqueous solution comprising bovine serum albumin (manufactured by Oriental Yeast Co., 1% by weight) and polyoxyethylene(10) octyl phenyl ether (manufactured by Wako Pure Chemical Industries, Ltd., 0.1% by weight), and saccharose (manufactured by Wako Pure Chemical Industries, Ltd., 1% by weight), for 10 minutes, and then dried at 40°C for 2 hours. Subsequently, a reverse side of the membrane, the side opposite to the antibody coating surface, was pasted together with a polyester film (100 µm in thickness) using spray paste.

At a position 0 to 8 mm from the upstream end of the antibody coating position was pasted together a polyester nonwoven fabric (dimensions: 6 mm × 8 mm, thickness: 2.5 mm).

Further, at a position 0 to 10 mm from the downstream end from the position at which the above polyester nonwoven fabric was pasted, a pulp nonwoven fabric (dimensions: 10 mm × 10 mm, thickness: 5 mm) was pasted together as a water-absorbent pad, to prepare a test strip. Six test strips were prepared as described above, and used for the following measurement.

### 2) Measurement

Each of test samples in which *E. coli* O157:H7 (manufactured by Kirkegaard & Perry Laboratories Inc.) was dispersed in a 0.1 M phosphate buffer (NaCl being contained at 0.9% by weight, pH: 7.4) at a concentration shown in Table 1 was prepared.

With the resulting test sample was separately mixed each of the latex particles immobilized with the goat anti-*E. coli* O157:H7 polyclonal antibody and the HRP prepared in Examples 1 to 3, and the blue colored latex particles immobilized with the anti-*E. coli* O157:H7 polyclonal antibody (without immobilizing the enzyme), so as to have each of the concentrations of the solid components of 0.02% by weight. Thereafter, 60 µl of each liquid mixture was separately added dropwise to the polyester nonwoven fabric portion of each test strip prepared in Item 1) above.

Each of the test strips used with the latex particles prepared in Examples 1 to 3, in which the antibody and the enzyme were both immobilized, was washed after 5 minutes by adding dropwise 60 µl of 0.1 M phosphate buffer to the same nonwoven fabric portion. After additional 5 minutes, 60 µl of TMB (3,3',5,5'-tetramethylbenzidine) membrane Peroxidase Substrate System (manufactured by Kirkegaard & Perry Laboratories Inc., pH 4.5) was added dropwise to the same nonwoven fabric portion. The presence or absence of color development after 10 minutes was visually observed.

On the other hand, to the test strip used with the blue colored latex particles-labeled anti-*E. coli* O157:H7 antibody prepared in Comparative Example 1, in which the enzyme was not immobilized, was added a liquid mixture of the test sample and the colored latex. The presence or absence of coloration was confirmed after 20 minutes.

The results are shown in Table 1. The criteria in. Table 1 are as follows.
- +:: Linear coloration is observed in the capture region.
- -:: No linear coloration is observed in the capture region.

**Table 1**

| | Concentration of Analyte (cells/ml) | | | | | |
|---|---|---|---|---|---|---|
| | 1×10⁵ | 1×10⁴ | 5×10³ | 1×10³ | 1×10² | 0 |
| Example 1 | + | + | + | + | - | - |
| Example 2 | + | + | + | + | - | - |
| Example 3 | + | + | + | + | - | - |
| Comparative Example 1 | + | - | - | - | - | - |

Although the number of cells of the level of 1 × 10⁵ cells/ml was required for the test in those without enzyme immobilization, i.e. the labeling with the colored latex, in the immunochromatography using the water-dispersible polymeric particles (enzyme-labeled specific conjugate) of the present invention in which both the enzyme and the antibody were immobilized, the *E. coli* O157:H7 could be detected at a concentration of the level of 1 × 10³ cells/ml.

In addition, in "Studies on Food Hygiene," Study Group Report of O157, the Ministry of Health and Welfare, **48**, 35 (1998), there have been reported that the detection sensitivity of O157 using conventional immunochromatographies (labeling with colored latex) or using enzyme immunochromatographies in which an enzyme used as a labeling agent was directly bound to an antibody is of the level of 10⁵ to 10⁶ cells/ml.

In the immunochromatographies using the enzyme-labeled specific conjugate of the present invention, a high sensitivity of about 100 to about 1,000 times that as compared to those of these conventional methods can be achieved, and the required period of time can be shortened.

### Example 5: Use of Test Strips for Immunochromatography

### 1) Preparation of Test Strips having Dried Reagent Region

There were mixed together 1 ml of a dispersion (2% by weight) of the latex particles immobilized with the goat anti-*E. coli* O157:H7 polyclonal antibody and the HRP prepared in Example 1, and 6 ml of a saccharose aqueous solution (10% by weight). With 10 µl of the resulting liquid mixture was impregnated rayon nonwoven fabric (dimensions: 6 mm × 8 mm), and the nonwoven fabric was dried at 30°C for 2 hours. This nonwoven fabric was pasted together to a position 12 to 20 mm from the opposite end of the antibody coating position on the side of the surface of the test strip having the capture region prepared in the same manner as in Item 1) of Example 4, to prepare a test strip having the capture region and the dried reagent region. Six test strips were prepared as described above, and used for the following measurement.

### 2) Measurement

To a polyester nonwoven fabric portion of the test strip obtained in Item 1) above was added dropwise 60 µl of the test sample used in Item 2) of Example 4, and 5 minutes thereafter, washing was carried out with adding dropwise 60 µl of 0.1 M phosphate buffer to the same nonwoven fabric portion. After additional 5 minutes, 60 µl of TMB (3,3',5,5'-tetramethylbenzidine) membrane Peroxidase Substrate System (manufactured by Kirkegaard & Perry Laboratories Inc., pH 4.5) was added dropwise to the same nonwoven fabric portion. The presence or absence of color development after 10 minutes was visually observed. As a result, in the same manner as in Example 4, the *E. coli* O157:H7 could be detected at a concentration of the level of 1 × 10³ cells/ml.

### Example 6: Preparation of Enzyme-Labeled Specific Conjugate (Enzyme-Antibody Immobilized Carrier)

Entirely the same procedures as in Example 1 were carried out except for using a goat anti-*Salmonella* polyclonal antibody (manufactured by Kirkegaard & Perry Laboratories Inc. at 1 mg/ml in 0.01 M-borate buffer at pH 8.2) when immobilizing the specific binding substance, to prepare latex particles immobilized with the goat anti-*Salmonella* polyclonal antibody and the HRP. The amount of the antibody immobilized was 12.3 mg, per 1 g of latex particles on a dry basis, and the amount of the enzyme immobilized was 2.3 mg, per 1 g of latex particles on a dry basis.

### Example 7: Preparation of Enzyme-Labeled Specific Conjugate (Enzyme-Antibody Immobilized Carrier)

Entirely the same procedures as in Example 1 were carried out except for using an anti-Vero toxin type 1-A subunit monoclonal antibody (manufactured by ViroStat at 1 mg/ml in 0.01 M-borate buffer at pH 8.2) when immobilizing the specific binding substance, to prepare latex particles immobilized with the anti-Vero toxin type 1-A subunit monoclonal antibody and the HRP. The amount of the antibody immobilized was 70 mg, per 1 g of latex particles on a dry basis, and the amount of the enzyme immobilized was 2 mg, per 1 g of latex particles on a dry basis.

### Example 8: Preparation of Enzyme-Labeled Specific Conjugate (Enzyme-Antibody Immobilized Carrier)

Entirely the same procedures as in Example 1 were carried out except for using an anti-Vero toxin type 2-A subunit monoclonal antibody (manufactured by ViroStat at 1 mg/ml in 0.01 M-borate buffer at pH 8.2) when immobilizing the specific binding substance, to prepare latex particles immobilized with the anti-Vero toxin type 2-A subunit monoclonal antibody and the HRP. The amount of the antibody immobilized was 68 mg, per 1 g of latex particles on a dry basis, and the amount of the enzyme immobilized was 1.8 mg, per 1 g of latex particles on a dry basis.

### Example 9: Detection of E. coli O157:H7 and Salmonella

### 1) Preparation of Test Strips

At a position 30 mm from one end of the nitrocellulose membrane (pore size: 8 µm, dimensions: 6 mm × 60 mm) was applied with a dispenser in two lines (capture region) 1.5 µl each of a goat anti-*E. coli* O157:H7 polyclonal antibody (at 1 mg/ml in 0.1 M-phosphate buffer at pH 7.4) and a goat anti-*Salmonella* polyclonal antibody (manufactured by Kirkegaard & Perry Laboratories Inc. at 1 mg/ml in 0.01 M-borate buffer at pH 7.4).

The resulting membrane was immersed in an aqueous solution comprising bovine serum albumin (manufactured by Oriental Yeast Co., 1% by weight) and polyoxyethylene(10) octyl phenyl ether (manufactured by Wako Pure Chemical Industries, Ltd., 0.1% by weight), and saccharose (manufactured by Wako Pure Chemical Industries, Ltd., 1% by weight), for 10 minutes, and then dried at 40°C for 2 hours. Subsequently, a reverse side of the membrane, the side opposite to the antibody coating surface, was pasted together with a polyester film (100 µm in thickness) using spray paste. At a position 0 to 8 mm from the upstream end of the antibody coating position was pasted together a polyester nonwoven fabric (dimensions: 6 mm × 8 mm, thickness: 2.5 mm). Further, at a position 0 to 10 mm from the downstream end from the position at which the above polyester nonwoven fabric was pasted, a pulp nonwoven fabric (dimensions: 10 mm × 10 mm, thickness: 5 mm) was pasted together as a water-absorbent pad, to prepare a test strip. Four test strips were prepared as described above, and used for the following measurement.

### 2) Measurement

Each of test samples in which *E. coli* O157:H7 (manufactured by Kirkegaard & Perry Laboratories Inc.) or *S. typhimurium* (manufactured by Kirkegaard & Perry Laboratories Inc.) was dispersed in a 0.1 M phosphate buffer (NaCl being contained at 0.9% by weight, pH: 7.4) at a concentration shown in Table 2 was prepared.

With the resulting test sample were separately mixed the latex particles immobilized with the goat anti-*E. coli* O157:H7 polyclonal antibody and the HRP prepared in Example 1, and the latex particles immobilized with the goat anti-*Salmonella* polyclonal antibody and the HRP prepared in Example 6, so as to have each of the concentrations of the solid components of 0.02% by weight. Thereafter, 60 µl of each liquid mixture was separately added dropwise to the polyester nonwoven fabric portion of each test strip prepared in Item 1) above.

Each of the test strips used with the latex particles prepared in Examples 1 and 6, in which the antibody and the enzyme were both immobilized, was washed after 5 minutes by adding dropwise 60 µl of 0.1 M phosphate buffer to the same nonwoven fabric portion. After additional 5 minutes, 60 µl of TMB (3,3',5,5'-tetramethylbenzidine) membrane Peroxidase Substrate System (manufactured by Kirkegaard & Perry Laboratories Inc., pH 4.5) was added dropwise to the same nonwoven fabric portion. The presence or absence of color development after 10 minutes was visually observed.

The results are shown in Table 2. The criteria in Table 2 are as follows.
- +:: Linear coloration is observed in the capture region.
- -:: No linear coloration is observed in the capture region.

**Table 2**

| | Concentration of Analyte (cells/ml) | | |
|---|---|---|---|
| E. coli O157:H7 | 1 × 10⁴ | 0 | 1 × 10⁴ |
| S. typhimurium | 0 | 1 × 10⁴ | 1 × 10⁴ |

| Criteria | | | |
|---|---|---|---|
| Line for Anti-E.coli O157:H7 | + | - | + |
| Line for Anti-Salmonella | - | + | + |

### Example 10: Detection of E. coli O157:H7 and Vero toxin

### 1) Preparation of Test Strips

At a position 30 mm from one end of the nitrocellulose membrane (pore size: 8 µm, dimensions: 6 mm × 60 mm) was applied with a dispenser in three lines (capture region) 1.5 µl each of a goat anti-*E. coli* O157:H7 polyclonal antibody (manufactured by Kirkegaard & Perry Laboratories Inc., at 1 mg/ml in 0.1 M-phosphate buffer at pH 7.4), an anti-Vero toxin type 1-B subunit monoclonal antibody (manufactured by ViroStat at 1 mg/ml in 0.01 M-borate buffer at pH 7.4), and an anti-Vero toxin type 2-B subunit monoclonal antibody (manufactured by ViroStat at 1 mg/ml in 0.01 M-borate buffer at pH 7.4).

The resulting membrane was immersed in an aqueous solution comprising bovine serum albumin (manufactured by Oriental Yeast Co., 1% by weight) and polyoxyethylene(10) octyl phenyl ether (manufactured by Wako Pure Chemical Industries, Ltd., 0.1% by weight), and saccharose (manufactured by Wako Pure Chemical Industries, Ltd., 1% by weight), for 10 minutes, and then dried at 40°C for 2 hours. Subsequently, a reverse side of the membrane, the side opposite to the antibody coating surface, was pasted together with a polyester film (100 µm in thickness) using spray paste. At a position 0 to 8 mm from the upstream end of the antibody coating position was pasted together a polyester nonwoven fabric (dimensions: 6 mm × 8 mm, thickness: 2.5 mm). Further, at a position 0 to 10 mm from the downstream end from the position at which the above polyester nonwoven fabric was pasted, a pulp nonwoven fabric (dimensions: 10 mm × 10 mm, thickness: 5 mm) was pasted together as a water-absorbent pad, to prepare a test strip. Eight test strips were prepared as described above, and used for the following measurement.

### 2) Measurement

Each of test samples in which *E. coli* O157:H7 (manufactured by Kirkegaard & Perry Laboratories Inc.), Vero toxin type 1 and Vero toxin type 2 (each being manufactured by DENKA SEIKEN Co.), was dispersed in a 0.1 M phosphate buffer (NaCl being contained at 0.9% by weight, pH: 7.4) at a concentration shown in Table 3 was prepared.

With the resulting test sample was separately mixed the latex particles immobilized with the goat anti-*E. coli* O157:H7 polyclonal antibody and the HRP prepared in Example 1, the latex particles immobilized with the anti-Vero toxin type 1-A subunit monoclonal antibody and the HRP prepared in Example 7, the latex particles immobilized with the anti-Vero toxin type 2-A subunit monoclonal antibody and the HRP prepared in Example 8, so as to have each of the concentrations of the solid components of 0.02% by weight. Thereafter, 60 µl of each liquid mixture was separately added dropwise to the polyester nonwoven fabric portion of each test strip prepared in Item 1) above.

Each of the test strips used with the latex particles prepared in Examples 1, 7 and 8, in which the antibody and the enzyme were both immobilized, was washed after 5 minutes by adding dropwise 60 µl of 0.1 M phosphate buffer to the same nonwoven fabric portion. After additional 5 minutes, 60 µl of TMB (3,3',5,5'-tetramethylbenzidine) membrane Peroxidase Substrate System (manufactured by Kirkegaard & Perry Laboratories Inc., pH 4.5) was added dropwise to the same nonwoven fabric portion. The presence or absence of color development after 10 minutes was visually observed.

The results are shown in Table 3. The criteria in Table 3 are as follows.
- +:: Linear coloration is observed in the capture region.
- -:: No linear coloration is observed in the capture region.

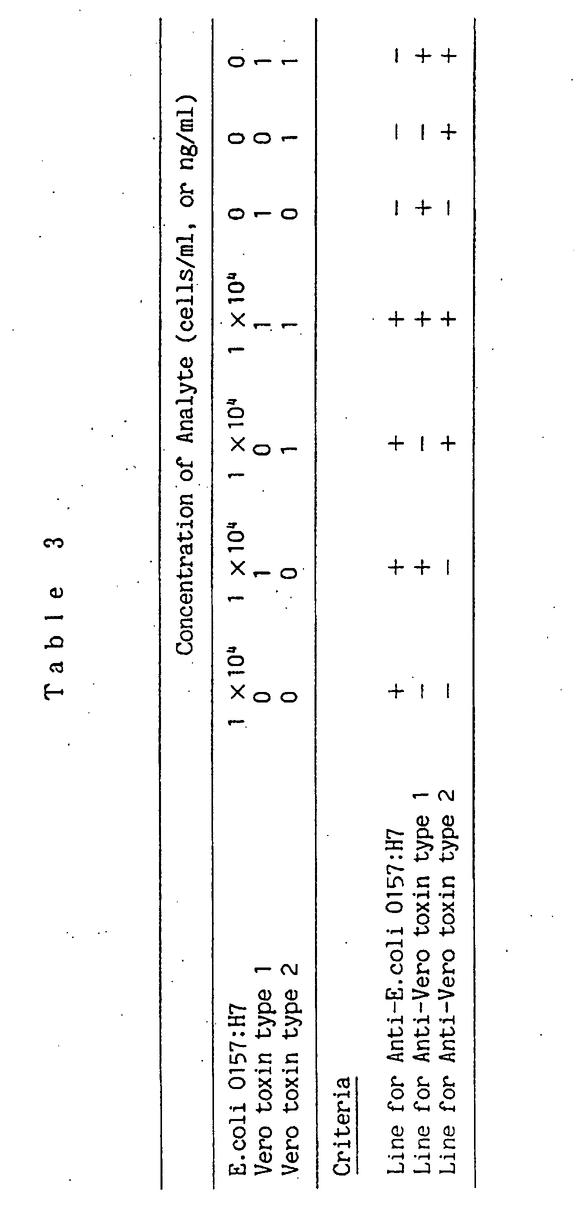

### Example 11: Detection of E. coli O157:H7 and Salmonella

Entirely the same procedures as in Example 1 were carried out except for using 2 ml each of a goat anti-*E. coli* O157:H7 polyclonal antibody (manufactured by Kirkegaard & Perry Laboratories Inc. at 1 mg/ml in 0.01 M-borate buffer at pH 8.2), and a goat anti-*Salmonella* polyclonal antibody (manufactured by Kirkegaard & Perry Laboratories Inc. at 1 mg/ml in 0.01 M-borate buffer at pH 8.2), to prepare latex particles immobilized with the goat anti-*E. coli* O157:H7 polyclonal antibody, the goat anti-*Salmonella* polyclonal antibody, and the HRP.

Entirely the same experiment as in Example 9 was carried out except for using the resulting latex particles prepared above, and it was found that *E. coli* O157:H7 and *Salmonella* could be detected.

### Example 12: Preparation of Test Strips for Immunochromatography and Use Thereof

### 1) Preparation of Test Strips Having Dried Reagent Region

There were mixed together 1 ml of a dispersion (2% by weight) of the latex particles immobilized with the goat anti-*E. coli* O157:H7 polyclonal antibody and the HRP prepared in Example 1, 1 ml of a dispersion (2% by weight) of the latex particles immobilized with the goat anti-*Salmonella* polyclonal antibody and the HRP prepared in Example 6, and 6 ml of a saccharose aqueous solution (10% by weight). With 10 µl of the resulting liquid mixture was impregnated a rayon nonwoven fabric was (dimensions: 6 mm × 8 mm), and the nonwoven fabric was dried at 30°C for 2 hours. This nonwoven fabric was pasted together to a position 12 to 20 mm from the opposite end of the antibody coating position on the side of the surface of the test strip having the capture region prepared in the same manner as in Item 1) of Example 9, to prepare a test strip having the capture region and the dried reagent region. Four test strips were prepared as described above, and used for the following measurement.

### 2) Measurement

To the polyester nonwoven fabric portion of each of the test strips prepared in Item 1) above was added dropwise 60 µl of the test sample used in Item 2) of Example 9, and washed after 5 minutes by adding dropwise 60 µl of 0.1 M phosphate buffer to the same nonwoven fabric portion. After additional 5 minutes, 60 µl of TMB (3,3',5,5'-tetramethylbenzidine) membrane Peroxidase Substrate System (manufactured by Kirkegaard & Perry Laboratories Inc., pH 4.5) was added dropwise to the same nonwoven fabric portion. The presence or absence of color development after 10 minutes was visually observed. As a result, *E. coli* O157:H7 and Salmonella could be simultaneously detected.

### Example 13: Preparation of Test Strips for Immunochromatography and Use Thereof

Entirely the same procedures as in Example 1 were carried out except for using 2 ml each of a goat anti-*E. coli* O157:H7 polyclonal antibody (manufactured by Kirkegaard & Perry Laboratories Inc. at 1 mg/ml in 0.01 M-borate buffer at pH 8.2), and a goat anti-*Salmonella* polyclonal antibody (manufactured by Kirkegaard & Perry Laboratories Inc. at 1 mg/ml in 0.01 M-borate buffer at pH 8.2), to prepare latex particles immobilized with the goat anti-*E. coli* O157:H7 polyclonal antibody, the goat anti-*Salmonella* polyclonal antibody, and the HRP. Entirely the same procedures as in Example 12 were carried out except for using the resulting latex particles prepared above to prepare a test strip having the capture region and the dried reagent region.

Entirely the same experiment as in Example 12 was carried out except for using the resulting test strip, and it was found that *E. coli* O157:H7 and *Salmonella* could be detected.

### Comparative example

### Example 14: Detection of E. coli O157:H7 and Salmonella by Directly Labeled Antibody with Enzyme

Entirely the same experiment as in Example 9 was carried out except for using an HRP-labeled goat anti-*E. coli* O157:H7 polyclonal antibody in place of the latex particles immobilized with the goat anti-*E. coli* O157:H7 polyclonal antibody and the HRP as prepared in Example 1, for using an HRP-labeled goat anti-*Salmonella* polyclonal antibody in place of the latex particles immobilized with the goat anti-*Salmonella* polyclonal antibody and the HRP prepared in Example 6, and for mixing the components so as to have each of the concentrations of the solid components of 0.001% by weight. As a result, *E. coli* O157:H7 and *Salmonella* could be detected in the same manner as in Example 9.

## Claims

1. A labeled conjugate comprising water-dispersible polymeric particles as a carrier, at least one immunochemical component selected from the group consisting of antigens, haptens, antibodies, and oligonucleotides, and a label wherein the label is an enzyme usable for detection selected from the group consisting of peroxidases, β-D-galactosidase, alkaline phosphatases, glucose oxidases, luciferase, esterases and β-D-glucuronidase, wherein the immunochemical component and the enzyme are linked via the carrier and each is immobilized onto a surface of the carrier, and wherein a total amount of the immunochemical component and the enzyme immobilized is from 5 to 200 mg per 1 g of the water-dispersible polymeric particles on a dry basis.

2. The labeled conjugate according to claim 1, wherein the immunochemical component and/or the enzyme are immobilized onto the surface of the carrier via a covalent bond.

3. The labeled conjugate according to claim 1 or 2 wherein the amount of the enzyme immobilized onto a surface of the carrier is from 1.0 to 100 mg per 1 g of the water-dispersible polymeric particles on a dry basis.

4. The labeled conjugate according to anyone of claims 1 to 3, wherein the enzyme is immobilized onto a surface of the carrier at a ratio of 0.6 to 60 mol per one mol of the immunochemical component immobilized onto the surface of the carrier.

5. The labeled conjugate according to any one of claims 1 to 4, wherein the enzyme is at least one enzyme selected from the group consisting of peroxidases and alkaline phosphatases.

6. The labeled conjugate according to any one of claims 1 to 5, wherein the enzyme activity is from 5,000 to 300,000 U, per 1 g of the water-dispersible polymeric particles on a dry basis, in a case where the enzyme to be immobilized onto the carrier is a peroxidase.

7. The labeled conjugate according to any one of claims 1 to 6, wherein a particle size of the water-dispersible polymeric particles is from 0.01 to 3 µm.

8. The labeled conjugate according to any one of claims 1 to 7, wherein each of the water-dispersible polymeric particles has a carboxyl group.

9. A method for detecting an analyte, comprising the steps of :
(1) forming a complex of:
(a) an enzyme-labeled specific conjugate resulting from linking via a carrier, an enzyme and a first specific binding substance capable of specifically binding to an analyte; and
(b) said analyte,
to wick the resulting complex on a water-absorbent substrate;
(2) capturing said complex at a capture region immobilized on the water-absorbent substrate with a second specific binding substance capable of specifically binding to the analyte; and
(3) detecting the captured complex.

10. A method for detecting a plurality of analytes, comprising the steps of:
(1) forming a complex or complexes of:
(a) an enzyme-labeled specific conjugate resulting from linking, via a carrier, an enzyme and a first specific binding substance capable of specifically binding to an analyte; and
(b) a plurality of different analytes,
to wick the resulting complex or complexes on a water-absorbent substrate;
(2) capturing said complex or complexes at the plurality of capture regions on the water-absorbent substrate, each of which is immobilized with a second specific binding substance capable of specifically binding to one of the plurality of different analytes; and
(3) detecting the captured complex or complexes.

11. The method according to claim 10, wherein said enzyme-labeled specific conjugate results from linking, via a carrier, an enzyme and a first specific binding substance capable of specifically binding to one of the plurality of different analytes, and a plurality of complexes are formed by using a plurality of different enzyme-labeled specific conjugates, each of which is capable of binding to one of the plurality of different analytes.

12. The method according to claim 11, wherein said enzyme-labeled specific conjugate results from linking, via a carrier, an enzyme and a plurality of first specific binding substances, each of which is capable of specifically binding to one of the plurality of the analytes.

13. The method according to any one of claims 9 to 12, wherein said enzyme-labeled specific conjugate is the labeled complex of any one of claims 1 to 8.

14. The method according to any one of claims 9 to 13, wherein said second specific binding substance is one or more substances selected from the group consisting of antibodies, antigens, haptens, and oligonucleotides.

15. The method according to any one of claims 9 to 14, wherein the second specific binding substance is immobilized on the water-absorbent substrate to form the capture region, and the resulting water-absorbent substrate is treated with a solution comprising a protein, a surfactant, and a sugar.

16. The method according to any one of claims 9 to 15, wherein the carrier is colored.

17. A test strip comprising:
(a) a capture region arranged on a water-absorbent substrate, comprising a second specific binding substance capable of specifically binding to an analyte, wherein the second specific binding substance is immobilized thereon;
(b) a dried reagent region comprising an enzyme-labeled specific conjugate resulting from linking, via a carrier, an enzyme and a first specific binding substance capable of specifically binding to the analyte, wherein the dried reagent region is arranged on the water-absorbent substrate such that the enzyme-labeled specific conjugate leaves and wicks therefrom when contacted with water; and
(c) a sample receiver arranged at a position upstream of the dried reagent region or the same position as the dried reagent region.

18. A test strip comprising:
(a) a plurality of capture regions arranged on a water-absorbent substrate, each comprising a second specific binding substance capable of specifically binding to one of a plurality of different analytes, wherein each of the second specific binding substances is immobilized thereon;
(b) a dried reagent region comprising an enzyme-labeled specific conjugate resulting from linking, via a carrier, an enzyme and first specific binding substances, each of which is capable of specifically binding to one of the plurality of analytes to form a complex, wherein the dried reagent region is arranged on the water-absorbent substrate such that the enzyme-labeled specific conjugate leaves and wicks therefrom when contacted with water; and
(c) a sample receiver arranged at a position upstream of the dried reagent region or the same position as the dried reagent region.

19. The test strip according to claim 18, wherein said enzyme-labeled specific conjugate results from linking, via a carrier, an enzyme and a first specific binding substance capable of specifically binding to one analyte, and wherein the dried reagent region comprises a plurality of different enzyme-labeled specific conjugates, each of which is capable of specifically binding to one of a plurality of different analytes.

20. The test strip according to claim 18, wherein said enzyme-labeled specific conjugate results from linking, via a carrier, an enzyme and a plurality of first specific binding substances, each of which is capable of specifically binding to one of a plurality of different analytes.

21. The test strip according to any one of claims 17 to 20, wherein said enzyme-labeled specific conjugate is the labeled complex of any one of claims 1 to 8.

22. The test strip according to any one of claims 17 to 21, wherein said second specific binding substance is one or more substances selected from the group consisting of antibodies, antigens, haptens, and oligonucleotides.

23. The test strip according to any one of claims 17 to 22, wherein the second specific binding substance is immobilized on the water-absorbent substrate to form the capture region, and the resulting water-absorbent substrate is treated with a solution comprising a protein, a surfactant, and a sugar.

24. The test strip according to any one of claims 17 to 23, wherein the carrier is colored.

25. A test kit of an analyte comprising:
(1) a test strip comprising a capture region arranged on a water-absorbent substrate, comprising a second specific binding substance capable of specifically binding to the analyte, and a sample receiver arranged at a position upstream of the capture region; and
(2) an enzyme-labeled specific conjugate resulting from linking, via a carrier, an enzyme and a first specific binding substance capable of specifically binding to the analyte.

26. A test kit of a plurality of analytes comprising:
(1) a test strip comprising a plurality of capture regions arranged on a water-absorbent substrate, each comprising a second specific binding substance capable of specifically binding to one of the plurality of different analytes, and a sample receiver arranged at a position upstream of the capture regions; and
(2) an enzyme-labeled specific conjugate resulting from linking, via a carrier, an enzyme and a first specific binding substance capable of specifically binding to the analyte.

## Patentansprüche

1. Markiertes Konjugat, umfassend in Wasser dispergierbare Polymerpartikel als Träger, mindestens eine immunchemische Komponente, ausgewählt aus der Gruppe bestehend aus Antigenen, Haptenen, Antikörpern und Oligonucleotiden, und eine Markierung, wobei die Markierung ein für den Nachweis verwendbares Enzym ist, ausgewählt aus der Gruppe bestehend aus Peroxidasen, β-D-Galactosidase, alkalischen Phosphatasen, Glucose-Oxidasen, Luciferase, Esterasen und β-D-Glucuronidase, wobei die immunchemische Komponente und das Enzym über den Träger miteinander verbunden sind und jedes auf einer Oberfläche des Trägers immobilisiert ist, und wobei die Gesamtmenge der immobilisierten immunchemischen Komponente und des immobilisierten Enzyms zwischen 5 und 200 mg pro 1 g der in Wasser dispergierbaren Polymerpartikel bezogen auf die Trockenmasse liegt.

2. Markiertes Konjugat nach Anspruch 1, wobei die immunchemische Komponente und/oder das Enzym über eine kovalente Bindung auf der Oberfläche des Trägers immobilisiert sind.

3. Markiertes Konjugat nach Anspruch 1 oder 2, wobei die Menge des auf einer Oberfläche des Trägers immobilisierten Enzyms von 1,0 bis 100 mg pro 1 g der in Wasser dispergierbaren Polymerpartikel bezogen auf die Trockenmasse beträgt.

4. Markiertes Konjugat nach einem der Ansprüche 1 bis 3, wobei das Enzym auf einer Oberfläche des Trägers in einem Verhältnis von 0,6 bis 60 Mol pro ein Mol der immunochemischen Komponente immobilisiert ist, die auf der Oberfläche des Trägers immobilisiert ist.

5. Markiertes Konjugat nach einem der Ansprüche 1 bis 4, wobei das Enzym mindestens ein Enzym ausgewählt aus der Gruppe bestehend aus Peroxidasen und alkalischen Phosphatasen ist.

6. Markiertes Konjugat nach einem der Ansprüche 1 bis 5, wobei die enzymatische Aktivität bei 5.000 bis 300.000 U pro 1 g der in Wasser dispergierbaren Polymerpartikel bezogen auf die Trockenmasse liegt, in einem Fall, in dem das auf dem Träger zu immobilisierende Enzym eine Peroxidase ist.

7. Markiertes Konjugat nach einem der Ansprüche 1 bis 6, wobei die Partikelgröße der in Wasser dispergierbaren Polymerpartikel bei 0,01 bis 3 µm liegt.

8. Markiertes Konjugat nach einem der Ansprüche 1 bis 7, wobei jedes der in Wasser dispergierbaren Polymerpartikel eine Carboxylgruppe hat.

9. Verfahren zum Nachweis eines Analyten, umfassend die folgenden Schritte:
(1) Bildung eines Komplexes aus:
(a) einem Enzym-markierten spezifischen Konjugat, erhalten durch Verbinden eines Enzyms und eines ersten spezifischen bindenden Stoffes, der in der Lage ist, spezifisch an einen Analyten zu binden, mittels eines Trägers; und
(b) dem Analyten,
wobei der resultierende Komplex auf einem Wasser absorbierenden Substrat eingesaugt wird;
(2) Binden des Komplexes an einer Bindungsregion, die mit einem zweiten spezifischen bindenden Stoff, der in der Lage ist, spezifisch an den Analyten zu binden, auf dem Wasser absorbierenden Substrat immobilisiert ist; und
(3) Nachweis des gebundenen Komplexes.

10. Verfahren zum Nachweis einer Vielzahl von Analyten, umfassend die folgenden Schritte:
(1 ) Bildung eines Komplexes oder von Komplexen aus:
(a) einem Enzym-markierten spezifischen Konjugat erhalten durch Verbinden eines Enzyms und eines ersten spezifischen bindenden Stoffes, der in der Lage ist, spezifisch an einen Analyten zu binden, mittels eines Trägers; und
(b) einer Vielzahl unterschiedlicher Analyten,
wobei der resultierende Komplex oder die Komplexe auf einem Wasser absorbierenden Substrat eingesaugt wird/werden;
(2) Binden des Komplexes oder der Komplexe an die Vielzahl der Bindungsregionen auf dem Wasser absorbierenden Substrat, von denen jede mit einem zweiten spezifischen bindenden Stoff immobilisiert ist, der in der Lage ist, spezifisch an einen aus der Vielzahl der unterschiedlichen Analyten zu binden; und
(3) Nachweis des oder der gebundenen Komplexe(s).

11. Verfahren nach Anspruch 10, wobei das Enzym-markierte spezifische Konjugat durch Verbinden eines Enzyms und eines ersten spezifischen bindenden Stoffes mittels eines Trägers erhalten wird, wobei der Stoff in der Lage ist, spezifisch an einen der Vielzahl unterschiedlicher Analyten zu binden, und eine Vielzahl von Komplexen durch die Verwendung einer Vielzahl verschiedener Enzym-markierter spezifischer Konjugate gebildet wird, von denen jedes in der Lage ist, einen aus der Vielzahl unterschiedlicher Analyten zu binden.

12. Verfahren nach Anspruch 11, wobei das Enzym-markierte spezifische Konjugat durch Verbinden eines Enzyms und einer Vielzahl erster spezifischer bindender Stoffe mittels eines Trägers erhalten wird, von denen jeder in der Lage ist, spezifisch an einen aus der Vielzahl der Analyten zu binden.

13. Verfahren nach einem der Ansprüche 9 bis 12, wobei das Enzym-markierte spezifische Konjugat der markierte Komplex nach einem der Ansprüche 1 bis 8 ist.

14. Verfahren nach einem der Ansprüche 9 bis 13, wobei der zweite spezifische bindende Stoff ein oder mehrere Stoffe ist, ausgewählt aus der Gruppe bestehend aus Antikörpern, Antigenen, Haptenen und Oligonucleotiden.

15. Verfahren nach einem der Ansprüche 9 bis 14, wobei der zweite spezifische bindende Stoff auf dem Wasser absorbierenden Substrat immobilisiert ist, um die Bindungsregion zu bilden, und das resultierende Wasser absorbierende Substrat mit einer Lösung behandelt wird, die ein Protein, einen grenzflächenaktiven Stoff und einen Zucker enthält.

16. Verfahren nach einem der Ansprüche 9 bis 15, wobei der Träger gefärbt ist.

17. Teststreifen, umfassend:
(a) eine Bindungsregion, angeordnet auf einem Wasser absorbierenden Substrat, umfassend einen zweiten spezifischen bindenden Stoff, der in der Lage ist, spezifisch an einen Analyten zu binden, wobei der zweite spezifische bindende Stoff darauf immobilisiert ist;
(b) eine getrocknete Reagens-Region, umfassend ein Enzym markiertes spezifisches Konjugat, das durch Verbinden eines Enzyms und eines ersten spezifischen bindenden Stoffes mittels eines Trägers erhalten wird, wobei der Stoff in der Lage ist, spezifisch an den Analyten zu binden, wobei die getrocknete Reagens-Region so auf dem Wasser absorbierenden Substrat angeordnet ist, dass das Enzym-markierte spezifische Konjugat bei Kontakt mit Wasser den Bereich verlässt und aufgesaugt wird; und
(c) einen Probenempfänger, der an einer stromaufwärts zur getrockneten Reagens-Region gelegenen Position oder an der gleichen Position wie die getrocknete Reagens-Region angeordnet ist.

18. Teststreifen, umfassend:
(a) eine Vielzahl von Bindungsregionen, angeordnet auf einem Wasser absorbierenden Substrat, wobei jede einen zweiten spezifischen bindenden Stoff umfasst, der in der Lage ist, spezifisch an eine Vielzahl unterschiedlicher Analyten zu binden, wobei jeder der zweiten spezifischen bindenden Stoffe darauf immobilisiert ist;
(b) eine getrocknete Reagens-Region, umfassend ein Enzym-markiertes spezifisches Konjugat, das durch Verbinden eines Enzyms und erster spezifischer bindender Stoffe mittels eines Trägers erhalten wird, wobei von den Stoffen jeder in der Lage ist, spezifisch an einen aus der Vielzahl der Analyten zu binden, um einen Komplex zu bilden, wobei die getrocknete Reagens-Region so auf dem Wasser absorbierenden Substrat angeordnet ist, dass das Enzym-markierte spezifische Konjugat bei Kontakt mit Wasser den Bereich verlässt und aufgesaugt wird; und
(c) einen Probenempfänger, der an einer stromaufwärts zur getrockneten Reagens-Region gelegenen Position oder an der gleichen Position wie die getrocknete Reagens-Region angeordnet ist.

19. Teststreifen nach Anspruch 18, wobei das Enzym-markierte spezifische Konjugat durch Verbinden eines Enzyms und eines ersten spezifischen bindenden Stoffes mittels eines Trägers erhalten wird, wobei der Stoff in der Lage ist, spezifisch an einen Analyten zu binden, und wobei die getrocknete Reagens-Region eine Vielzahl unterschiedlicher Enzym-markierter spezifischer Konjugate umfasst, von denen jedes in der Lage ist, spezifisch an einen aus einer Vielzahl unterschiedlicher Analyten zu binden.

20. Teststreifen nach Anspruch 18, wobei das Enzym-markierte spezifische Konjugat durch Verbinden eines Enzyms und einer Vielzahl erster spezifischer bindender Stoffe mittels eines Trägers erhalten wird, wobei von den Stoffen jeder in der Lage ist, spezifisch an einen aus einer Vielzahl unterschiedlicher Analyten zu binden.

21. Teststreifen nach einem der Ansprüche 17 bis 20, wobei das Enzym-markierte spezifische Konjugat der markierte Komplex nach einem der Ansprüche 1 bis 8 ist.

22. Teststreifen nach einem der Ansprüche 17 bis 21, wobei der zweite spezifische bindende Stoff ein oder mehrere Stoffe ist, ausgewählt aus der Gruppe bestehend aus Antikörpern, Antigenen, Haptenen und Oligonucleotiden.

23. Teststreifen nach einem der Ansprüche 17 bis 22, wobei der zweite spezifische bindende Stoff auf dem Wasser absorbierenden Substrat immobilisiert ist, um die Bindungsregion zu bilden, und das resultierende Wasser absorbierende Substrat mit einer Lösung, die ein Protein, einen grenzflächenaktiven Stoff und einen Zucker umfasst, behandelt wird.

24. Teststreifen nach einem der Ansprüche 17 bis 23, wobei der Träger gefärbt ist.

25. Kit zum Testen auf einen Analyten, umfassend:
(1) einen Teststreifen, umfassend eine Bindungsregion, angeordnet auf einem Wasser absorbierenden Substrat, umfassend einen zweiten spezifischen bindenden Stoff, der in der Lage ist, spezifisch an den Analyten zu binden, und einen Probenempfänger, der an einer stromaufwärts zur Bindungsregion gelegenen Position angeordnet ist; und
(2) ein Enzym markiertes spezifisches Konjugat, das durch Verbinden eines Enzyms und eines ersten spezifischen bindenden Stoffes mittels eines Trägers erhalten wird, wobei der Stoff in der Lage ist, spezifisch an den Analyten zu binden.

26. Kit zum Testen auf eine Vielzahl von Analyten, umfassend:
(1) einen Teststreifen, umfassend eine Vielzahl von Bindungsregionen, die auf einem Wasser absorbierenden Substrat angeordnet sind, wobei jede einen zweiten spezifischen bindenden Stoff umfasst, der in der Lage ist, spezifisch an einen aus der Vielzahl der Analyten zu binden, und einen Probenempfänger, der an einer stromaufwärts zur Bindungsregion gelegenen Position angeordnet ist; und
(2) ein Enzym-markiertes spezifisches Konjugat, das durch Verbinden eines Enzyms und eines ersten spezifischen bindenden Stoffes mittels eines Trägers erhalten wird, wobei der Stoff in der Lage ist, spezifisch an den Analyten zu binden.

## Revendications

1. Conjugué marqué comprenant des particules polymériques dispersibles dans l'eau en tant que support, au moins un composant immunochimique choisi dans le groupe constitué des antigènes, haptènes, anticorps et oligonucléotides, et un marqueur, dans lequel le marqueur est une enzyme utilisable pour la détection choisie dans le groupe constitué de péroxydases, β-D-galactosidase, phosphatases alkalines, glucose oxydases, luciférase, estérases et β-D-glucuronidase ; le composant immunochimique et l'enzyme étant liés par le support et chacun étant immobilisé sur une surface du support, et dans lequel une quantité totale du composant immunochimique et de l'enzyme immobilisés est de 5 à 200 mg pour 1g de particules polymériques dispersibles dans l'eau, sur une base sèche.

2. Conjugué marqué selon la revendication 1 dans lequel le composant immunochimique et/ou l'enzyme sont immobilisés à la surface du support par l'intermédiaire d'une liaison covalente.

3. Conjugué marqué selon la revendication 1 ou 2 dans lequel la quantité d'enzyme immobilisée à une surface du support est de 1,0 à 100 mg pour 1 g de particules polymériques dispersibles dans l'eau sur une base sèche.

4. Conjugué marqué selon l'une des revendications 1 à 3 dans lequel l'enzyme est immobilisée à une surface du support à un taux de 0,6 à 60 moles par mole du composant immunochimique immobilisé à la surface du support.

5. Conjugué marqué selon l'une des revendications 1 à 4 dans lequel l'enzyme est au moins une enzyme choisie dans le groupe constitué de peroxydases et phosphatases alcalines.

6. Conjugué marqué selon l'une des revendications 1 à 5, dans lequel l'activité enzymatique est de 5 000 à 300 000 U pour 1 g de particules polymériques dispersibles dans l'eau sur une base sèche, dans le cas où l'enzyme à immobiliser sur le support est une peroxydase.

7. Conjugué marqué selon l'une des revendications 1 à 6, dans lequel une taille particulaire des particules polymériques dispersibles dans l'eau est de 0,01 à 3µm.

8. Conjugué marqué selon l'une des revendications 1 à 7, dans lequel chacune des particules polymériques dispersibles dans l'eau a un groupe carboxyle.

9. Méthode de détection d'un analyte, comprenant les étapes de :
(1) formation d'un complexe entre :
(a) un conjugué spécifique marqué avec une enzyme résultant de la liaison par l'intermédiaire d'un support d'une enzyme et d'une première substance de liaison spécifique capable de se lier spécifiquement à un analyte ; et
(b) ledit analyte,
pour faire pénétrer par capillarité le complexe résultant sur un substrat absorbant l'eau ;
(2) capture dudit complexe en une région de capture immobilisée sur le substrat absorbant l'eau avec une seconde substance de liaison spécifique capable de se lier spécifiquement à l'analyte ; et
(3) détection du complexe capturé.

10. Méthode de détection d'une pluralité d'analytes, comprenant les étapes de :
(1) formation d'un complexe ou de complexes entre :
(a) un conjugué spécifique marqué avec une enzyme résultant de la liaison, par l'intermédiaire d'un support, d'une enzyme et d'une première substance de liaison spécifique capable de se lier spécifiquement à un analyte ; et
(b) une pluralité d'analytes différents,
pour faire pénétrer par capillarité le complexe ou les complexes résultant sur un substrat absorbant l'eau ;
(2) capture dudit complexe ou desdits complexes en la pluralité des régions de capture sur le substrat absorbant l'eau, chacun d'entre eux étant immobilisé avec une seconde substance de liaison spécifique capable de se lier spécifiquement à l'un de la pluralité d'analytes différents; et
(3) détection du ou des complexes capturés.

11. Méthode selon la revendication 10, dans laquelle ledit conjugué spécifique marqué avec une enzyme résulte de la liaison, par l'intermédiaire d'un support, d'une enzyme et d'une première substance de liaison spécifique capable de se lier spécifiquement à l'un de la pluralité d'analytes différents; et une pluralité de complexes sont formés par utilisation d'une pluralités de conjugués spécifiques marqués avec une enzyme, chacun d'entre eux étant capable de se lier à l'un de la pluralité d'analytes différents.

12. Méthode selon la revendication 11, dans laquelle ledit conjugué spécifique marqué avec une enzyme résulte de la liaison, par l'intermédiaire d'un support, d'une enzyme et d'une pluralité de premières substances de liaison spécifiques, chacune étant capable de se lier spécifiquement à l'un de la pluralité d'analytes.

13. Méthode selon l'une quelconque des revendications 9 à 12, dans laquelle ledit conjugué spécifique marqué avec une enzyme est le complexe marqué selon l'une quelconque des revendications 1 à 8.

14. Méthode selon l'une quelconque des revendications 9 à 13, dans laquelle ladite seconde substance de liaison spécifique est une ou plusieurs substances choisies dans le groupe constitué d'anticorps, antigènes, haptènes et oligonucléotides.

15. Méthode selon l'une quelconque des revendications 9 à 14, dans laquelle ladite seconde substance de liaison spécifique est immobilisée sur le substrat absorbant l'eau pour former la région de capture, et le substrat absorbant l'eau résultant est traité avec une solution comprenant une protéine, un tensio-actif et un sucre.

16. Méthode selon l'une quelconque des revendications 9 à 15, dans laquelle ledit support est coloré.

17. Languette de test comprenant :
(a) une région de capture disposée sur un substrat absorbant l'eau, comprenant une seconde substance de liaison spécifique capable de se lier spécifiquement à un analyte, dans laquelle la seconde substance de liaison spécifique est immobilisée à la surface de celle-ci ;
(b) une région de réactif sèche comprenant un conjugué spécifique marqué avec une enzyme résultant de la liaison par l'intermédiaire d'un support d'une enzyme et d'une première substance de liaison spécifique capable de se lier spécifiquement à l'analyte, dans laquelle la région de réactif sèche est disposée sur le substrat absorbant l'eau de sorte que le conjugué spécifique marqué avec une enzyme migre et pénètre par capillarité lorsqu'il est en contact avec de l'eau ; et
(c) un récepteur d'échantillon disposé en une position aval par rapport à la région de réactif sèche ou en une position identique à celle de la région de réactif sèche.

18. Languette de test comprenant :
(a) une pluralité de régions de capture disposées sur un substrat absorbant l'eau, chacune comprenant une seconde substance de liaison spécifique capable de se lier spécifiquement à l'un d'une pluralité d'analytes différents, dans laquelle chacune des secondes substances de liaison spécifiques est immobilisée à la surface de celles-ci ;
(b) une région de réactif sèche comprenant une conjugué spécifique marqué avec une enzyme résultant de la liaison par l'intermédiaire d'un support d'une enzyme et de premières substances de liaison spécifiques, chacune étant capable de se lier spécifiquement à l'un de la pluralité d'analytes pour former un complexe, dans laquelle la région de réactif sèche est disposée sur le substrat absorbant l'eau de sorte que le conjugué spécifique marqué avec une enzyme migre et pénètre par capillarité lorsqu'il est en contact avec de l'eau ; et
(c) un récepteur d'échantillon disposé en une position aval par rapport à la région de réactif sèche ou en une position identique à celle de la région de réactif sèche.

19. Languette de test selon la revendication 18, dans laquelle ledit conjugué spécifique marqué avec une enzyme résulte de la liaison, par l'intermédiaire d'un support, d'une enzyme et d'une première substance de liaison spécifique capable de se lier spécifiquement à un analyte, et dans laquelle la région de réactif sèche comprend une pluralité de conjugués spécifiques marqués avec une enzyme différents , chacun étant capable de se lier spécifiquement à l'un d'une pluralité d'analytes différents.

20. Languette de test selon la revendication 18, dans laquelle ledit conjugué spécifique marqué avec une enzyme résulte de la liaison par l'intermédiaire d'un support d'une enzyme et d'une pluralité de premières substances de liaison spécifiques, chacune étant capable de se lier spécifiquement à l'un d'une pluralité d'analytes différents.

21. Languette de test selon l'une des revendications 17 à 20, dans laquelle ledit conjugué spécifique marqué avec une enzyme est le complexe marqué selon l'une quelconque des revendications 1 à 8.

22. Languette de test selon l'une quelconque des revendications 17 à 21, dans laquelle ladite seconde substance de liaison spécifique est une ou plusieurs substances choisies dans le groupe constitué d'anticorps, antigènes, haptènes et oligonucléotides

23. Languette de test selon l'une quelconque des revendications 17 à 22, dans laquelle ladite seconde substance de liaison spécifique est immobilisée sur le substrat absorbant l'eau pour former la région de capture, et le substrat absorbant l'eau résultant est traité avec une solution comprenant une protéine, un tensio-actif et un sucre.

24. Languette de test selon l'une quelconque des revendications 17 à 23, dans laquelle ledit support est coloré.

25. Kit de test d'un analyte comprenant :
(1) une languette de test comprenant une région de capture disposée sur un substrat absorbant l'eau, comprenant une seconde substance de liaison spécifique capable de se lier spécifiquement a l'analyte, et un récepteur d'échantillon disposé en une position aval par rapport à la région de capture ; et
(2) un conjugué spécifique marqué avec une enzyme résultant de la liaison, par l'intermédiaire d'un support, d'une enzyme et d'une première substance de liaison spécifique capable de se lier spécifiquement a l'analyte.

26. Kit de test d'une pluralité d'analytes comprenant :
(1) une languette de test comprenant une pluralité de régions de capture disposées sur un substrat absorbant l'eau, chacune comprenant une seconde substance de liaison spécifique capable de se lier spécifiquement à l'un de la pluralité d'analytes différents, et un récepteur d'échantillon disposé en une position aval par rapport aux régions de capture ; et
(2) un conjugué spécifique marqué avec une enzyme résultant de la liaison, par l'intermédiaire d'un support, d'une enzyme et d'une première substance de liaison spécifique capable de se lier spécifiquement à l'analyte.
